(19) Europäisches Patentamt
European Patent Office
Office européen des brevets

(11) **EP 1 494 575 B1**

(12) **EUROPÄISCHE PATENTSCHRIFT**

(45) Veröffentlichungstag und Bekanntmachung des Hinweises auf die Patenterteilung:
**19.09.2007 Patentblatt 2007/38**

(21) Anmeldenummer: 03711777.7

(22) Anmeldetag: **17.04.2003**

(51) Int Cl.:
***A61B 3/10*** *(2006.01)*

(86) Internationale Anmeldenummer:
**PCT/CH2003/000257**

(87) Internationale Veröffentlichungsnummer:
**WO 2003/086180 (23.10.2003 Gazette 2003/43)**

(54) **MESSUNG OPTISCHER EIGENSCHAFTEN**

MEASUREMENT OF OPTICAL PROPERTIES

MESURE DE PROPRIETES OPTIQUES

(84) Benannte Vertragsstaaten:
**CH DE FR GB LI**

(30) Priorität: **18.04.2002 CH 6532002**

(43) Veröffentlichungstag der Anmeldung:
**12.01.2005 Patentblatt 2005/02**

(73) Patentinhaber: **HAAG-STREIT AG**
**3098 Köniz (CH)**

(72) Erfinder: **WÄLTI, Rudolf**
**CH-3097 Liebefeld (CH)**

(74) Vertreter: **Roshardt, Werner Alfred**
**Keller & Partner**
**Patentanwälte AG**
**Schmiedenplatz 5**
**Postfach**
**3000 Bern 7 (CH)**

(56) Entgegenhaltungen:
| | |
|---|---|
| **WO-A-01/38820** | **WO-A-96/35100** |
| **US-A- 4 315 672** | **US-A- 5 301 010** |
| **US-A- 5 856 883** | **US-A- 6 137 585** |
| **US-B1- 6 256 102** | |

## Beschreibung

### Technisches Gebiet

Die Erfindung betrifft einen opthalmologischen Untersuchungs- und/oder Behandlungsplatz gemäss Patentanspruch 1.

### Stand der Technik

Bei ophthalmologischen Untersuchungs- und Behandlungsplätzen, wie beispielsweise bei einer Foto-Spaltlampe 900 P-BQ der Firma Haag-Streit AG oder einer in der EP-A 0 916 306 beschriebenen Spaltlampe können einzelne Elemente, wie eine Linsentrageinheit, ein Mikroskop, ein Beleuchtungsoberteil, ... ausgetauscht werden.

In der WO 01/19303 ist ein Verfahren zur Fotoablation auf der Kornea mit einem Laserstrahl sowie eine Vorrichtung hierzu beschrieben. Es werden hintereinander folgende Teilablationsvorgänge vorgenommen, wobei nach jedem Teilablationsvorgang die Dicke der Kornea mit einer mit einem Michelson-Interferometer zusammenarbeitenden Messeinrichtung bestimmt wird. Die Messeinrichtung kann mit einem Steromikroskop zusammenarbeiten, wobei hier die optische Achse des Stereomikroskops und die Achse des Behandlungsstrahlenganges zusammenfallen. Die Lage des für die Ablation vorgesehenen Bereichs wird ermittelt und eine Freigabe zur Ablation nur gegebenen, sofern der Bereich sich innerhalb vorgegebener Toleranzwerte befindet.

In der US 5,856,883 ist ein Operationsmikroskop beschrieben, an das lediglich die Strahlung externer Strahlungsquellen über Lichtleiter ankoppelbar ist.

In der US 5,301,101 ist ein Michelson-Interferometer beschrieben, welches mit einer kurzkohärenten Freiraumstrahlung einer Strahlungsquelle arbeitet. In einem Strahlarm des Interferometers sind zwei einen Laufzeitunterschied hervorrufende Reflektoren vorhanden.

In der US 5,493,109 ist ein chirurgisches Mikroskop für ophthalmologische Anwendungen beschrieben. An das Mikroskop ist eine Messanordnung für eine optische Kohärenztomographie angeschlossen. Das Mikroskop hat eine sich automatische einstellende Fokuslinse. Eine Videokamera ist ferner an das Mikroskop anschliessbar.

Aus der EP-A 0 932 021 ist eine Vorrichtung mit einem Laser-Interferometer zur Ermittlung der Ebenheit einer Oberfläche bekannt. In der bekannten Vorrichtung wurde ein Laserstrahl durch einen Strahlteiler in zwei Strahlen aufgeteilt. Diese beiden Strahlen wurden mit optischen Ablenkmitteln unter einem vorgegebenen Winkel parallel gerichtet. Die beiden parallelen Strahlen trafen auf ein auf einem Halter angeordnetes Strahlumlenkelementepaar (Prismen). Jedes dieser Umlenkelemente lenkte jeden Strahl derart um, dass er seitlich versetzt, aber parallel zum einfallenden Strahl reflektiert wurde. Jeder der reflektierten Strahlen wurde auf je einen Reflektor gesandt. Die Reflektoren waren ortsfest mit dem Strahlteiler verbunden. Jeder der auf die Reflektoren treffenden Strahlen wurde in sich selbst reflektiert und nach einer weiteren Rückreflektion durch die Strahlumlenkelemente durch den Strahlteiler vereinigt und interferierend in einen Detektor eingestrahlt. Wurde nun der Halter bewegt, so änderte sich das Interferenzmuster im Detektor, wodurch die Ebenheit einer Oberfläche ermittelbar war.

Die bekannte Vorrichtung war aufwendig in ihrem optischen Aufbau und gestattete lediglich die Bestimmung der Ebenheit einer Oberfläche.

### Aufgabe der Erfindung

Aufgabe der Erfindung ist es nicht bei einem ophthalmologischen Gerät einige Untereinheiten, welche eventuell serviceanfällig sind, austauschbar anzuordnen, sondern einen ophthalmologischen Untersuchungs- und Behandlungsplatz zu schaffen, der vielseitig, vorzugsweise durch eine einfache Modifikation, verwendbar ist und insbesondere voluminöse Anordnungen vor dem Patientenauge vermeidet. Mit diesem Untersuchungs- und Behandlungsplatz sollen "in-vivo"-Messungen von Abständen, Dicken, Oberflächenverläufen,..., welche Messungen an unterschiedlichen Orten in einem Gegenstand beinhalten, optimal, d. h. mit einer Messfehlerminimierung durchführbar sein.

### Lösung der Aufgabe

Diese Aufgabe wird dadurch gelöst, dass der ophthalmologische Untersuchungs- und/oder Behandlungsplatz modular aufgebaut wird, d. h. eine Reihe auswechselbarer Einheiten aufweist. Aufgrund dieser Modularität ist der Untersuchungs- und/oder Behandlungsplatz derart auf- und umbaubar, dass er den Platz lediglich eines Gerätes beansprucht, aber es erlaubt, die Funktionalität unterschiedlicher Einzelgeräte zu erreichen. Der modulare Aufbau besteht aus einer Beleuchtungseinheit, einer Beobachtungseinheit, einer Auswerteeinheit und einer Messanordnung sowie einem unmittelbar vor dem Patientenauge anbringbaren Patientenmodul. Messanordnung und Beleuchtungseinheit sind oftmals voluminös ausgebildet oder erzeugen für den Patienten störende Wärme oder Lüftungsströme. Sie sind hier

EP 1 494 575 B1

vom Patienten entfernt angeordnet und über optische Fasern mit dem Patientenmodul verbunden. Die Verbindung der optischen Fasern am Patientenmodul ist lösbar ausgebildet. Aufgrund dieser Lösbarkeit können problemlos unterschiedliche Messanordnungen und auch Beleuchtungseinheiten, je nachdem, welche Untersuchungen oder Beobachtungen vorgenommen werden sollen, angeschlossen werden. Der Anschluss erfolgt über Faserstecker (-koppler). Im Patientenmodul befindet sich dann anschliessend an die Faserkoppler lediglich eine Kollimatoroptik, welche das aus jeweils einer Faser austretende Strahlungssignal in eine Freiraumstrahlung verwandelt bzw. auf die Faserenden auftreffende Strahlungssignale in diese einkoppelt.

Der ophthalmologische Untersuchungs- und Behandlungsplatz wird nun unter Verwendung einer Messanordnung als modulares Teileelement derart ausgebildet, dass er zum Messen optischer Eigenschaften wenigstens zweier voneinander distanzierter Bereiche bei einem transparenten und/oder diffusiven Gegenstand sowie zur Dicken-, Abstands- und/oder Profilmessung verwendbar ist. Die Dicken-, Abstands- und/oder Profilmessung wird mittels Kurzkohärenzreflektometrie vorgenommen. Wird als Gegenstand ein Auge verwendet, so handelt es sich um einen ophthalmologischen Untersuchungs- und Behandlungsplatz; es können aber auch beliebige transparente und/oder diffusive Gegenstände ausgemessen werden.

Die Transparenz von Gegenständen hängt von deren wellenlängenabhängigen Schwächungskoeffizienten $\alpha$ [$cm^{-1}$] und deren Dicke bzw. der vorgegebenen Messstrecke d ab. Als transparent werden Gegenstände bezeichnet, deren Transmissionsfaktor T = exp ($-\alpha \cdot d$) noch innerhalb des Messbereichs der unten beschriebenen Interferometer liegt, wobei bei den unten beschriebenen Interferometern infolge des "Hin- und Rückweges" der Strahlung die Transmission $T^2$ ist. In diffusiven Gegenständen wird die Strahlung stark gestreut, nicht notwendigerweise absorbiert. Beispielsweise sind als diffusive Gegenstände Milchglasscheiben, Delrin, organische Gewebe (Haut, menschliche und tierische Organe, Pflanzenteile etc.) anzusehen.

Eine Kurzkohärenzreflektometrie wurde in der Regel für präzise, rasche und nichtinvasive Abbildungen vorgenommen. Typischerweise wurde in einer optischen Anordnung mit einem Michelson-Interferometer der Strahl einer Strahlungsquelle in einen Referenz- und einen Messstrahl mit einem Strahlteiler aufgespalten. In der Regel wurde eine Strahlungsquelle mit einer kurzen Kohärenzlänge ausgewählt. Eine Aufspaltung in Referenz- und Messstrahl sowie deren Wiedervereinigung erfolgte mit einem Strahlteiler und bei Verwendung von Faseroptikwegen mit einem Faserkoppler. Die optische Weglängenänderung im Referenzarm konnte durch Verschieben eines Referenzspiegels auf einer Translationsbühne erreicht werden. Vorteilhafterweise verwendete man jedoch einen rotierenden transparenten Würfel, wie er in der WO96/35100 beschrieben war. Nur wenn der Weglängenunterschied kleiner als die Kohärenzlänge der Strahlung der Strahlungsquelle war, entstand nach der Wiedervereinigung des reflektierten Referenz- und Messstrahls ein Interferenzmuster. Das Interferenzmuster wurde auf einen Photodetektor gebracht, welcher die Strahlungsintensität während der Veränderung der Spiegelposition mass. Da die Frequenz der Strahlung des reflektierten Referenzstrahls wegen der Spiegelverschiebung eine Dopplerverschiebung erfuhr, konnte, wie unten skizziert, das Interferenzsignal mit elektronischen Mitteln, wie beispielsweise in der WO 99/22198 beschrieben, durch Erhöhung des Signal-Rauschverhältnisses ausgewertet werden.

Messfehler traten jedoch bei der WO 99/22198 auf, wenn Abstände, welche wenigstens zwei Messvorgänge notwendig machten, in optisch transparenten bzw. eine optische Strahlung diffus transmittierenden Gegenständen gemessen werden sollten, und die Gegenstände über dem gesamten Messzyklus innerhalb der geforderten Messtoleranz nur schwer oder nicht ausreichend fixiert werden konnten. Diese Probleme traten insbesondere bei "in-vivo"-Messungen auf.

Um "in-vivo"-Messungen von Abständen, Dicken, Oberflächenverläufen,..., welche Messungen an unterschiedlichen Orten in einem Gegenstand beinhalten, optimal, d. h. mit einer Messfehlerminimierung durchzuführen, werden die optischen Eigenschaften wenigstens zweier voneinander distanzierter Bereiche bei einem transparenten und/oder diffusiven Gegenstand bzw. einem Auge mit einer Messzeit im Subsekundenbereich ermittelt. Hierzu wird eine "Michelson-artige" Anordnung verwendet, mit der die von einer Strahlungsquelle ausgehende kurzkohärente Strahlung in eine Mess- und eine Referenzstrahlung aufgeteilt wird. Die Messstrahlung bestrahlt die Bereiche. Der Referenzstrahlung wird eine Laufzeitänderung aufgeprägt und an wenigstens zwei einen Laufzeitunterschied hervorrufenden Reflektoren reflektiert. Die reflektierte Referenzstrahlung wird dann interferierend mit der reflektierten Messstrahlung vereinigt. Die vereinigte Strahlung wird detektiert und das detektierte Signal zur Distanzmessung ausgewertet.

Zur Messung optischer Eigenschaften mit einer Messzeit im Subsekundenbereich (notwendig für eine "in-vivo"-Messung) wenigstens zweier voneinander distanzierter Bereiche bei einem transparenten und/oder diffusiven Gegenstand, wie zur Abstands-, Längen-, Dicken- und Profilmessung notwendig, wird der Gegenstand mit einer der Anzahl Bereiche entsprechenden Anzahl Messstrahlen gleichzeitig oder kurz nacheinander bestrahlt. Durch den Ausdruck "bei" einem Gegenstand soll ausgedrückt werden, dass die Bereiche sich an Orten im Gegenstand sowie auch auf dem Gegenstand, z.B. seitlich versetzt befinden können. Die Messstrahlen, welche unterschiedliche Laufzeiten haben, interferieren mit Referenzstrahlen, welche ebenfalls bis auf eine Bestimmungstoleranz unterschiedliche Laufzeiten haben.

Der Laufzeitunterschied im Referenzstrahlengang entspricht einem optischen Abstand zweier Raumpunkte (Bereiche) in Bezug auf die Ausbreitungsrichtung der Messstrahlung, wobei wenigstens einer der Raumpunkte wenigstens geringfügig (typischerweise mindestens $10^{-4}$% der Strahlungsintensität) reflektiert. Die Messstrahlen können somit übereinander liegen (Dicken-, Distanz-, Längenmessung), parallel zueinander verlaufen (Oberflächenprofil, ...) oder beliebige Winkel zueinander aufweisen (Dicken-, Distanzmessung, ... bei vorgegebenem Winkel zu einer Referenzfläche).

Zur Erzeugung der Laufzeitänderung der Referenzstrahlung, welche vorzugsweise periodisch erfolgt, sind mehrere Verfahren möglich. Es kann hierzu beispielsweise, wie oben bereits ausgeführt, ein rotierender "Würfel" mit teilweise verspiegelten Seitenflächen, wie in der WO 96/35100 beschrieben, verwendet werden. Es können aber auch die Reflektoren linear, vorzugsweise periodisch, verschoben werden. Der in der WO 96/35100 beschriebene "Würfel" ergibt eine Laufzeitänderung, welche periodisch und nahezu über den gesamten Verlauf linear erfolgt. Die linear bewegten Spiegel ergeben hingegen aufgrund der vorzunehmenden Beschleunigungen bei den Richtungsänderungen keine linearen Laufzeitänderungen.

Im Referenzarm wird nun gegenüber einem "landläufigen" Michelson-Interferometer nicht mehr mit nur einem reflektierten Strahl gearbeitet, sondern je nach Anzahl der auszumessenden Bereiche mit mehreren Strahlreflexionen. Diese Strahlreflexionen wird man vorteilhaft derart ausbilden, dass die Teilstrahlen immer in sich selbst zurückreflektiert werden, was jedoch nicht zwingend ist. Eine derartige optische Anordnung ist einfach auszuführen.

Um diese mehreren Strahlreflexionen zu erreichen, können nun mehrere nebeneinander in Strahlrichtung versetzte Spiegel als sogenannter Stufenspiegel angeordnet werden. Man kann nun den Stufenspiegel gesamthaft mit dem Referenzstrahl beleuchten oder die einzelnen Spiegel nacheinander. Wird z. B. der bereits oben erwähnte "Würfel" verwendet, so erfolgt durch diesen eine seitliche Strahlablenkung, so dass ein Spiegel nach dem anderen bei der Würfelrotation getroffen wird. Es kann aber auch eine rotierende oder eine linear über die Spiegel bewegte Blende verwendet werden. Weitere Varianten sind unten beschrieben.

Um vorzugsweise eine hohe räumliche Auflösung zu erhalten, wird man die Messstrahlung auf die auszumessenden Bereiche fokussieren. Ausführungsbeispiele sind ebenfalls unten beschrieben.

Nach der Vornahme der Wegdifferenz bzw. -differenzen erfolgt vorzugsweise zur Dickenmessung eine Vereinigung der Messstrahlen zu einer einzigen Strahlkonfiguration mit einer einzigen optischen Achse. Auch kann die Strahlkonfiguration über den Gegenstand, insbesondere periodisch, bewegt werden. Es ergibt sich hierbei ein seitliches Abscannen. Dieses Abscannen mit einem Abspeichern der ermittelten Werte kann zur Erstellung von Profilen dienen. Anstatt die beiden Messstrahlen entlang einer optischen Achse zu fokussieren, können auch jeweils wenigstens zwei Messstrahlen in einem Abstand nebeneinander verlaufen und fokussiert werden, um ein Oberflächenprofil zu ermitteln.

Die Messstrahlen haben im Vergleich zu den Bereichsabständen, insbesondere zu den Bereichsabständen ausgehend von einem Referenzort, eine kurze Kohärenzlänge. Die Messstrahlen können ferner jeweils sich voneinander unterscheidende Strahlungsfrequenzen haben. Es müssen dann jedoch mehrere Strahlungsquellen verwendet werden. Man kann auch mit nur einer Strahlungsquelle arbeiten und über Filter eine Aufteilung vornehmen. Hierbei ergibt sich jedoch ein Verlust an Breitbandigkeit; auch müssen einige der Komponenten mit einer aufwendigen Beschichtung versehen werden.

Anstelle unterschiedlicher Strahlungsfrequenzen bzw. ergänzend hierzu, können die Messstrahlen sich voneinander unterscheidende Polarisationszustände haben, was einen einfacheren Aufbau ergibt. Vorzugsweise wird man auch eine Fokussierung der Messstrahlen in den auszumessenden Bereich bzw. die auszumessenden Bereiche vornehmen. Da mit einer "Michelson-Interferometer"-artigen optischen Anordnung gearbeitet wird, können die augenblicklichen Positionen der reflektierenden Elemente im Referenzarm als Referenzorte dienen. Es kann nun die tatsächliche Position hierzu verwendet werden oder ein mit dem Referenzort gekoppelter anderer Wert, wie beispielsweise die Verdrehungsposition des in der WO 96/35100 beschriebenen rotierenden Würfels.

Die Messung wird an einem in den Messarm einbringbaren, optisch transparenten und/oder diffusiven Gegenstand durchgeführt. Anstelle eines optisch transparenten und/oder diffusiven Gegenstands kann auch mit einem Gegenstand gearbeitet werden, dessen Oberfläche stark reflektierend ist. Bei einem reflektierenden Gegenstand kann mit der erfindungsgemässen Methode insbesondere dessen Oberflächenprofil ermittelt werden. Der Gegenstand kann jedoch optisch transparent und/oder diffusiv sein und eine (wenigstens einige wenige Prozente) reflektierende Oberfläche haben. In diesem Fall können dann sowohl Oberflächen, wie auch Dicken bzw. deren Verläufe ermittelt werden.

Neben im Gegenstand "hintereinander" liegenden Bereichen (Orte) zur Dickenmessung können selbstverständlich auch "nebeneinander" liegende Bereiche (Orte) zur Bestimmung von Oberflächenkrümmungen bzw. Oberflächenverläufen ausgemessen werden.

Die Versetzung der Reflektoren wird näherungsweise derart eingestellt, dass sie einem zu erwartenden Messergebnis einer zu bestimmenden Dicke, eines Abstands, ... bis auf eine Bestimmungstoleranz entspricht. Mit der Wegvariationseinheit im Referenzarm muss dann nur noch der nicht bekannte (zu bestimmende) Anteil der Dicke, des Abstands etc. ermittelt werden. Soll z. B. die tatsächliche Länge eines menschlichen Auges bestimmt werden, so weiss man bereits schon vorher, dass Augen eine optische Länge von 34 mm mit einer Längentoleranz von +/- 4 mm aufweisen. Es kann nun hier eine Versetzung auf 34 mm eingestellt und mit der Wegvariationseinheit eine Variation von lediglich 8 mm vorgenommen werden.

Mit der unten beschriebenen Vorrichtung (Anordnung) und deren Ausführungsvarianten kann am Auge neben der Augenlänge (zentral, peripher), die Vorderkammertiefe (zentral, peripher), die Corneadicke (zentral, peripher), die Linsendicke (zentral, peripher) und die Glaskörpertiefe sowie entsprechende Oberflächenprofile (Topographie) der Corneavorderfläche, Cornearückfläche, Linsenvorderfläche, Linsenrückfläche und der Retina gemessen werden. Ferner lassen sich hiermit die Krümmungsradien z. B. der Corneavorderfläche, der Cornearückfläche, der Linsenvorderfläche und der Linsenrückfläche bestimmen. Hierzu wird der für die Augenoberfläche als Gegenstandsoberfläche bestimmte Messstrahl "irgendwo" zwischen der Corneavorderseite und der Linsenrückseite fokussiert. Durch diesen "Kompromiss" kann dann die Reflexion an der Corneavorderseite, der Cornearückseite, der Linsenvorderseite und der Linsenrückseite detektiert werden. Die Distanz zwischen der Cornearückseite und der Linsenvorderseite ist dann die Vorderkammertiefe. Bedingung für diese Messung ist jedoch, dass der "optische" Hub (ca. 8 mm) der Wegvariationseinheit derart gross ist, dass von der Corneavorderseite bis zur Linsenrückseite gescannt werden kann.

Eine einzige Messung verarbeitet somit die Reflexionen an mehren Bereichen nahezu gleichzeitig. Um die einzelnen Reflexionen messtechnisch dennoch unterscheiden zu können, haben die Messstrahlen unterschiedliche optische Eigenschaften, wie unterschiedliche Polarisationsrichtung, unterschiedliche Wellenlänge, .... Man kann jedoch auch mit nicht unterscheidbaren Strahlen arbeiten und durch eine Veränderung der Versetzung der Reflektoren die beiden Interferenzsignale zur Deckung bringen. In diesem Fall ist dann die eingestellte Versetzung gleich dem gesuchten Abstand, der Dicke etc.. Die Verwendung von nicht unterscheidbaren Strahlen führt zu einem Sensitivitätsverlust.

Je nach Anzahl verwendeter Messstrahlen können ein oder mehrere Abstände mit einer Messung bestimmt werden. Die Weglängenänderungen im Referenzarm können, wie in der WO 96/35100 beschrieben, mit einem rotierenden transparenten Würfel vor einem feststehenden Reflektor vorgenommen werden. Ein derartiger Würfel kann problemlos mit über 10 Hz rotieren. D.h. bei den meisten Messungen kann der auszumessende Gegenstand, ohne dass besondere Vorkehrungen für seine Fixierung vorgesehen werden, als in Ruhe befindlich betrachtet werden.

Am Patientenmodul wird man vorzugsweise ein Anzeigeelement anordnen, welches über eine lösbare elektrische Signalleitung mit der Auswerteeinheit verbunden ist. Auf dem Anzeigeelement können dann Messergebnisse, Behandlungsanweisungen, ... für den Arzt angezeigt werden.

Die Beobachtungseinheit kann nun derart ausgebildet werden, dass sie Teil des Patientenmoduls ist. D.h. der Arzt hält das Patientenmodul vor das Patientenauge oder setzt es auf dessen Oberfläche auf und schaut durch es hindurch auf bzw. in das Auge.

Es kann aber auch eine elektronische Beobachtungseinheit mit auswertbaren Bildsignalen vorgesehen werden. Diese ist mit einem im Patientenmodul angeordneten Okular und einem Objektiv zur Augenbetrachtung ausgebildet.

Die Beobachtungseinheit hat dann ein im Patientenmodul angeordnetes Bildaufnahmeelement (CCD) und eine einen zu betrachtenden Augenbereich auf einem Bildaufnahmeelement abbildende Optikeinheit. Die Optikeinheit ist ebenfalls im Patientenmodul angeordnet. Bildaufnahmeelement und Optikeinheit können auch doppelt und gegeneinander distanziert ausgebildet sein, um eine Stereobetrachtung zu ermöglichen. Das Bildaufnahmeelement ist dann über eine elektrische Signalleitung mit der entfernten Auswerteeinheit verbunden. Mit der Bildaufnahmeinheit aufgenommene Bilder können auch auf dem oben erwähnten Anzeigeelement, welches am Patientenmodul angeordnet bzw. in diesem integriert ist, wiedergegeben werden.

Das Patientenmodul kann man mit einem Gehäuse versehen, welches in seinen Abmessungen etwa einem handelsüblichen Kontaktglas, eventuell mit einem etwas grösseren Querschnitt (Volumenbedarf), nachempfunden ist. Die räumliche Ausbildung des Patientenmoduls sollte jedoch möglichst klein sein und vor dem Patientenauge lediglich einen kleinen Raumbereich beanspruchen. Voluminöse Elemente vor dem Auge rufen in der Regel ein Unwohlsein des Patienten hervor. Man kann aber auch als Halteeinrichtung einen Handgriff oder eine Ausrichteinheit anbringen. Mit dieser Ausrichteinheit kann dann eine Positionierung des Patientenmoduls in Bezug auf das Auge vorgenommen werden.

Die Mess- und/oder Beobachtungseinheit kann mit einer Auswerteeinheit zur Auswertung von Messdaten verbunden werden, wobei die Auswerteeinheit vorzugsweise computergestützt arbeiten sollte. Die Auswerteeinheit kann auch über ein Datennetz mit anderen, abrufbare Daten aufweisenden Datenspeichern verbunden sein, um die ermittelten und/oder ausgewerteten Daten mit den anderen Daten verarbeiten zu können. Hierdurch sind gute Diagnosemöglichkeiten gegeben, da Werte und Informationen aus Datenbanken abgerufen werden können.

Weitere Ausführungsvariationen zur Erfindung und deren Vorteile ergeben sich aus dem nachfolgenden Text. Allgemein sei bemerkt, dass die nachfolgend mit Strahlteiler bezeichneten optischen Einheiten eine Strahlteilung aber auch ein Zusammenfügen von zwei Strahlen vornehmen können.

**Kurze Beschreibung der Zeichnungen**

Nachfolgend werden Beispiele des erfindungsgemässen ophthalmologischen Untersuchungs- und/oder Behandlungsplatzes, der erfindungsgemässen Messanordnung, mit der das erfindungsgemässe Verfahren durchführbar ist, anhand von Zeichnungen näher erläutert. Es zeigen:

Fig. 1     ein Blockschaltbild eines erfindungsgemässen modularen, ophthalmologischen Untersuchungs- und/oder Behandlungsplatzes, u.a. mit einer Messanordnung,

Fig. 2     eine Ausführungsvariante eines vor dem Patientenauge zu platzierenden Patientenmoduls des in **Figur 1** dargestellten Untersuchungs- und/oder Behandlungsplatzes,

Fig. 3     eine weitere Variante zu dem in **Figur 3** dargestellten Patientenmodul,

Fig. 4     ein optisches Blockschaltbild einer beispielsweisen Ausführung einer Messanordnung, wie sie vorzugsweise im in **Figur 1** als Blockschaltbild gezeigten Untersuchungs- und/oder Behandlungsplatz einsetzbar ist,

Fig. 5     eine Variante zur Reflektoranordnung im Referenzarm des in **Figur 4** dargestellen optischen Aufbaus der in **Figur 4** verwendbaren Messanordnung,

Fig. 6     eine weitere Variante zur Reflektoranordnung im Referenzarm analog zu **Figur 5**,

Fig. 7     eine Variante zu der in **Figur 4** dargestellten Messanordnung,

Fig. 8     eine Seitenansicht der in **Figur 7** verwendeten Prismenanordnung in der dortigen Blickrichtung V,

Fig. 9     eine Variante zu den in den **Figuren 4** und **7** gezeigten Messanordnungen,

Fig. 10    einen Messstrahlenverlauf zur Profilermittlung unmittelbar vor dem auszumessenden Gegenstand,

Fig. 11    eine Variante zu den in den **Figuren 4, 7 und 9** gezeigten Messanordnungen mit mehreren Messstrahlen.

Fig. 12    eine vergrösserte Darstellung des Messstrahlenverlaufs der in **Figur 11** dargestellten Messanordnung im Bereich des auszumessenden Gegenstands (z.B. Auge),

Fig. 13    ein optisches Blockschaltbild einer Variante der Messanordnung, bei der die Strahlung grösstenteils in optischen Fasern verläuft, wobei hier beispielsweise die Augenoberfläche zur Darstellung der Auftrefforte der Strahlen um 90° verdreht gezeichnet ist,

Fig. 14    eine schematische Darstellung eines Stereomikroskops eines Spaltlampengeräts mit einem Messstrahlengang im Mittelkanal des Mikroskops und

Fig. 15    ein Spaltlampengerät mit einem auf das Mikroskop aufsteckbaren Adapter.

## Wege zur Ausführung der Erfindung

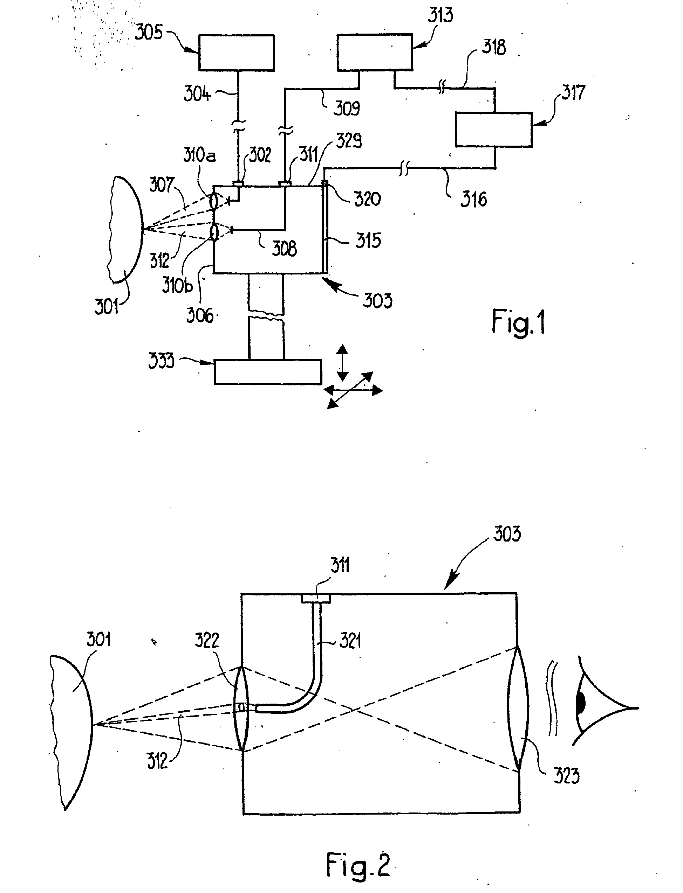

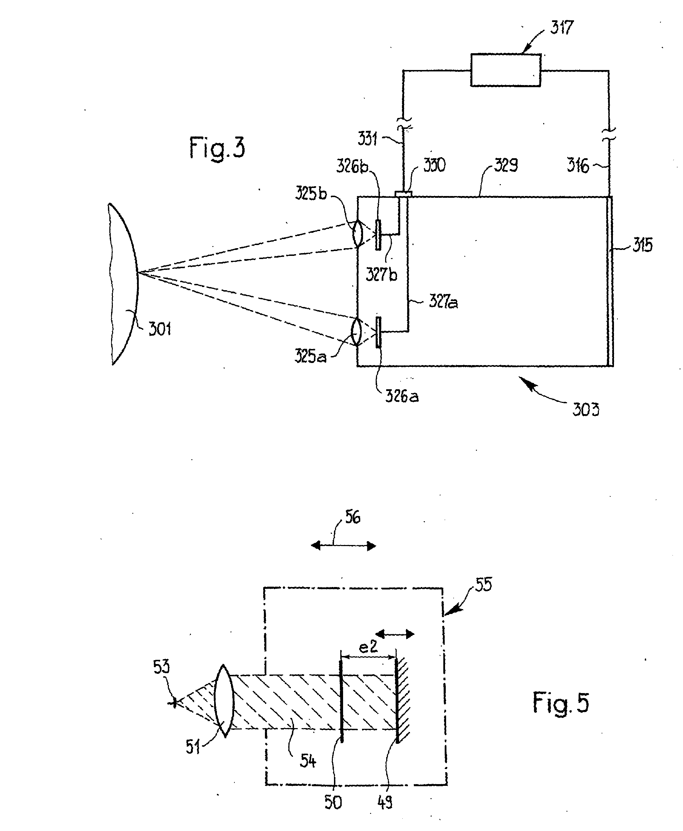

Der in **Figur 1** in einer Ausführungsvariante in einem "Blockschaltbild" dargestellte ophthalmologische Untersuchungs- und/oder Behandlungsplatz ist in einem modularen Aufbau ausgeführt. In unmittelbarer Nähe vor einem Patientenauge **301** ist ein Patientenmodul **303** positionierbar. Mit dem Patientenmodul **303** ist über eine mittels einer Faserkupplung **302** lösbare optische Faser **304** eine Beleuchtungseinheit **305** verbunden. In der Beleuchtungseinheit **305** ist eine nicht dargestellte Strahlungsquelle angeordnet, deren Strahlung über die Faser **304** in das Patientenmodul geführt und von dieser dann mit einer Kollimatorlinse **310a** als Freiraumstrahlung **307** auf bzw. in das Auge **301** gestrahlt wird. Im Patientenmodul **303** ist eine Beobachtungseinheit angeordnet, die unten beschrieben und in den **Figuren 2 und 3** schematisch gezeigt ist.

Das Patientenmodul **303** arbeitet mit einer unten beschriebenen Messanordnung zusammen. Die Messanordnung hat eine optische Faser **309**, die hier Teil eines Messarms einer "Michelson-Interferometer"-artigen Messanordnung ist. Die Faser **309** ist ebenfalls mittels eines Kupplungssteckers **311** mit dem Patientenmodul **303** lösbar verbunden. Die Strahlung der Faser **309** wird vom Patientenmodul **303** als Freiraumstrahlung **312** in bzw. auf das Auge **301** gerichtet. Die Freiraumstrahlung **312** wird mit einer Kollimatorlinse **310b** erzeugt. Die Kollimatorlinse **310b** ist vor dem Ende einer Faser **308** angeordnet, die von der Faserkupplung **311** in der Wand **329** des Patientenmoduls **303** bis zur dem Patientenauge **301** benachbarten Gehäusewand **306** verläuft.

Alle restlichen Komponenten der Messanordnung sind entfernt vom Patientenmodul 303 angeordnet, wobei die Anordnung mit den restlichen Komponenten symbolisch als Gebilde **313** gekennzeichnet ist.

An der dem Auge **301** abgewandten Seite des Patientenmoduls **303** ist ein Anzeigeelement **315** angeordnet.

Dieses Anzeigeelement **315** ist mittels elektrischer Kupplung **320** und einer elektrischen Verbindung **316** signalmässig mit einer Auswerteeinheit **317** lösbar verbunden. Die Auswerteeinheit **317** ist über eine weitere elektrische Signalleitung **318** mit dem Gebilde **313** verbunden.

Die Betrachtung des Auges **310** kann nun, wie in **Figur 2** dargestellt, direkt erfolgen. Ausgehend von der Faserkupplung **311** im Messarm wird hier eine weitere Faser **321** durch die Objektivlinse **322** für die direkte Beobachtung geführt. Am zur Kupplung **311** entgegengesetzten Ende der Faser **321** ist dann eine Kollimatorlinse **323** angeordnet, welche die Freiraumstrahlung **312** auf den gewünschten Bereich im bzw. am Auge **301** fokussiert. Die Freiraumstrahlung für die Beleuchtung ist der Übersichtlichkeit wegen in **Figur 2** weggelassen.

Anstelle einer direkten Betrachtung kann auch eine Betrachtung mittels elektronischer Hilfsmittel erfolgen, wie in **Figur 3** dargestellt ist. **Figur 3** zeigt eine stereoskopische Betrachtung mit zwei optischen Systemen **325a** und **325b,** deren Abbildungen eines Augenbereiches auf je ein Bildaufnahmeelement (z. B. CCD) **326a** bzw. **326b** fallen. Die elektrischen Signalausgänge **327a** und **327b** gehen auf eine in der Gehäusewand **329** des Patientenmoduls **303** angeordnete elektrische Kupplung **330,** an der lösbar ein Signalkabel **331** zur Auswerteeinheit **317** steckt. Das in der Auswerteeinheit **317** gegebenenfalls bearbeitete Bild kann dann über die Verbindung **316** zur Darstellung auf das Anzeigeelement **315** gesandt werden.

Die Strahlung der Beleuchtungseinheit **305** kann über eine eigene optische Faser **304** zum Patientenmodul **303** geführt werden. Sie kann aber auch vorzugsweise im Gebilde **313** in die Faser **309** eingekoppelt werden.

Das Patientenmodul **303** wird mit einer Halteeinrichtung **333** vor dem Patientenauge **301** positioniert. Die Halteeinrichtung kann nun ein Handgriff sein odereine Verstelleinrichtung, welche gesteuert horizontal und in der Höhe eine Ortsveränderung erlaubt.

Das Patientenmodul **303** wird man möglichst klein ausbilden, um den Patienten in seinem Augenbreich nicht durch voluminöse Einheiten zu beunruhigen. Ein ideales Volumen wäre etwa die Grösse handelsüblicher Kontaktgläser. Die Einheit wird jedoch aufgrund der zu installierenden Kollimatorlinsen etwas grösser ausfallen.

Aufgrund des modularen Aufbaus des Untersuchungs- und/oder Behandlungplatzes kann dieser lediglich den Platz eines einzigen Gerätes beanspruchen und die Funktionalität unterschiedlicher Einzelgeräte aufweisen sowie zusätzlich neben seiner Vielseitigkeit lediglich eine kleine, das Wohlbefinden des Patienten in keiner Weise störende Einheit vor dem Auge besitzen.

In **Figur 4** ist ein Ausführungsbeispiel für eine Messanordnung mit einer "Michelson-Interferometer"-artigen optischen Anordnung dargestellt. Diese Messanordnung kann bevorzugt zusammen mit dem oben erwähnten Patientenmodul **303** in einem modularen Mess- und Behandlungsaufbau eingesetzt werden. In der optischen Messanordnung werden weitgehend faseroptische Komponenten verwendet, welche eine grosse räumliche Flexibilität sowie ein Arbeiten in einer verhältnismässig rauhen Umgebung gestatten. In den nachfolgend beschriebenen Ausführungsbeispielen werden zum leichteren Verständnis lediglich zwei Bereiche **2a** und **2b** im auszumessende Gegenstand **1,** hier einem Auge, im Messarm **7** ausgemessen. Lediglich unmittelbar vor dem Messobjekt **1,** hier einem Auge, und vor der Spiegelanordnung **3** im Referenzarm **5** wird mit einer Freiraumstrahlung **6a** und **6b** gearbeitet. Die optische Anordnung der Messanordnung hat neben dem Referenzarm **5** einen Messarm **7,** in dem der auszumessenden Gegenstand **1** angeordnet ist. Eine Strahlungsquelle **9** sendet eine kurzkohärente Strahlung aus, welche in einer optischen Faser **10** zu einem Faserkoppler **11** geführt wird. Die Kohärenzlänge der Strahlung ist kürzer gewählt als die unten beschriebenen, auszumessenden Abstände im Gegenstand **1.** Als Strahlungsquelle **9** kann beispielsweise eine Superlumineszensdiode oder eine andere breitbandige Strahlungsquelle (Licht-) verwendet werden. Der von der Strahlungsquelle **9** ausgehende und in der Faser **10** geführte sogenannte Quellenstrahl wird vom Faserkoppler **11** in einen Referenz- und einen Messstrahl aufgeteilt. Der Messstrahl verläuft nach dem Faserkoppler **11** in einer optischen Faser **13** mit einem fasertechnischen Polarisationskontroller **15.** Am dem Faserkoppler **11** abgewandten Ende **16** der Faser **13** tritt dann die Messstrahlung als Freiraumstrahlung **6b** aus. Die austretende Freiraumstrahlung **6b** wird mit einem Linsensystem **17** auf bzw. in die beiden Messbereiche **2a** und **2b** fokussiert. Je nach Distanz zu den Messbereichen kann die Freiraumstrahlung **6b** zu einem parallelen Strahl kollimiert und dann auf die beiden Messbereiche **2a** und **2b** fokussiert oder, wie in **Figur 4** dargestellt, direkt in die Bereiche **2a** und **2b** fokussiert werden.

**Figur 4** dient zur Ermittlung der Augenlänge. Die Freiraumstrahlung **6b** wird hier beispielsweise mit einer ersten Fokussierlinse **19** des Linsensystems **17** auf den Messbereich **2b** auf der Retina **20** fokussiert. Das Linsensystem **17** weist eine weitere Fokussierlinse **21** auf, welche von der Fokussierlinse **17** in Richtung auf das Auge **1** zu distanziert angeordnet ist. Der zentrale Bereich der Linse **21** weist einen Durchbruch **23** auf, durch den die auf den Bereich **2b** fokussierte Strahlung ungehindert passieren kann. Die Randbereiche **24** der Linse **21** fokussieren dann die durch die Linse **19** "vorfokussierte" Strahlung auf den Messbereich **2a** auf der Hornhautvorderfläche **25.**

Die "Lochlinse" **21** wird man vorzugsweise in der Ausbreitungsrichtung des Messstrahls **6b** verschiebbar ausbilden. Hierdurch ist gewährleistet, dass auch bei einer Fehlsichtigkeit (z.B. Myopie oder Hyperopie) des zu untersuchenden Auges **1** der Messstrahl wenigstens näherungsweise auf die Retina **20** fokussierbar ist.

Anstelle der Anordnung mit einer "Lochlinse" kann auch ein diffraktives Element verwendet werden.

Der Referenzarm **5** hat ausgehend vom Faserkoppler **11** ebenfalls eine mit diesem verbundene Faser **27,** an deren dem Faserkoppler **11** abgewandtem Ende **29** die Freiraumstrahlung **6a** austritt. Der Referenzarm **5** enthält ferner eine Anordnung **3** von mehreren Reflektoren, welche die auf sie fallende Freiraumstrahlung **6a** in sich selbst zurückreflektieren. Die einzelnen Reflektoren sind derart gegeneinander versetzt, dass die auf sie fallenden Strahlungen gegeneinander im Referenzarm **5** einen Laufzeitunterschied erhalten. In dem hier gezeigten Beispiel sind nur zwei Reflektoren **31a** und **31b** vorhanden, da lediglich eine Distanz $d_1$ zweier Bereiche **2a** und **2b** im Gegenstand 1 (Messobjekt Auge) ermittelt werden soll. Sollen mehrere Bereiche zusammen ausgemessen werden, müssen selbstverständlich entsprechend viele Reflektoren angeordnet werden. Ein Versatz $d_2$ der beiden Reflektoren **31a** und **31b** entspricht einem bis auf eine Toleranz zu erwartenden Distanzwert $d_1$ der beiden Bereiche **2a** und **2b** im Auge 1.

Die aus dem Faserende **29** austretende Freiraumreferenzstrahlung **6a** wird mit einer Kollimatorlinse **33** soweit aufgeweitet, dass beide Reflektoren **31a** und **31b** ausgeleuchtet werden können. Im kollimierten Strahlengang **34** nach der Linse **33** ist eine rotierende Blende **35** angeordnet, welche derart ausgebildet ist, dass einmal der Reflektor **31a** und dann der Reflektor **31b** bestrahlt wird. Auf diese rotierende Blende **35** kann verzichtet werden. Man kann sie aber einsetzen, um eine eindeutige Zuordnung zu den Messsignalen zu erreichen. Es könnte nämlich sein, dass die Reflexionseigenschaften des vorderen und des hinteren Messbereichs **2a** und **2b** nahezu identisch sind. Es ist dann nicht immer möglich zu entscheiden, ob das erste, mit einem Photodetektor **37** detektierte, durch eine interferierende Überlagerung im Faserkoppler **11** entstandene Messsignal vom vorderen Messbereich **2a** oder vom hinteren Messbereich **2b** stammt. Beim Auge ist die Eindeutigkeit normalerweise ohne die Verwendung einer solchen Blende **35** möglich, da sich die Messsignale vom vorderen Augenabschnitt (Cornea, Vorderkammer, Linse) und der Retina **20** in eindeutiger Art unterscheiden.

Beide Reflektoren **31a** und **31b** sind in der Art eines Stufenspiegels auf einer Basis **39** jedoch gegeneinander verstellbar, wie durch einen Doppelpfeil **40** angedeutet, angeordnet. Die Basis **39** ist periodisch wie durch den anderen Doppelpfeil **41** angedeutet, senkrecht zum einfallenden Referenzfreiraumstrahl **6a** bewegbar. Alle Reflektoren **31a** und **31b** sind hochreflektierend und zueinander parallel liegend ausgebildet. Die Basis **39** kann z. B. eine vibrierende Lautsprechermembran sein.

Soll die Augenlänge bestimmt werden, so werden die beiden Reflektoren **31a** und **31b** in einem Abstand $d_2$ von der zu erwartenden typischen Augenlänge von 34 mm (Toleranz $\pm$ 4mm) angeordnet. Die periodische Bewegung der Reflektoranordnung **3** d.h. der Basis **39** gemäss Doppelpfeil **41** erfolgt dann mit einigen Schwingungen pro Minute (z.B. mit 10 Hz). Immer wenn die optischen Weglängen im Referenzarm 5 und im Messarm 7 zwischen dem Faserkoppler **11** und Reflektor **31a** sowie dem Faserkoppler **11** und dem Messbereich **2a** oder zwischen dem Faserkoppler **11** und dem Reflektor **31b** sowie dem Faserkoppler **11** und dem Messbereich **2b** gleich lang sind, detektiert der Detektor **37** ein Interferenzsignal. Da die Auslenkung der Basis **39** bekannt ist, kann somit die Augenlänge $d_1$ ermittelt werden.

Soll eine andere Distanz $d_1$ ermittelt werden, werden die beiden Reflektoren **31a** und **31b** auf einen anderen gegenseitigen Abstand $d_2$ eingestellt und dann die Basis **39** periodisch hin und her bewegt. Bei der Einstellung des Abstands $d_2$ ist lediglich darauf zu achten, dass die Einstelltoleranz im Hubbereich der Basis **39** liegen muss, da ansonsten kein Interferenzsignal erhalten wird.

Der grosse Vorteil der Anordnung bei einem Einsatz in der Ophthalmologie liegt insbesondere darin, dass vor dem Auge des Patienten nur die Linsenanordnung **17** vorhanden ist. Auch sind keinerlei bewegte Teile vorhanden. Die Linsenanordnung **17** lässt sich klein und handlich ausbilden. Sie kann z. B. in einem zylinderartigen Handgriff untergebracht werden. Auch die beiden Linsen **19** und **21** der Linsenanordnung **17** wird man einstellbar ausbilden, um eine Anpassung an der Fokussierung auf die entsprechenden auszumessenden Bereiche vornehmen zu können. Die Einstellmöglichkeit der beiden Linsen **19** und **21** ist in **Figur 4** durch die beiden Doppelpfeile **43a** und **43b** angedeutet. Sollen mehr als zwei Bereiche auf einmal ausgemessen werden, so sind dementsprechend mehr Fokussierlinsen anzuordnen. Wobei ausgehend vom Faserende **16** die erste Linse analog zur Linse **19** voll ausgebildet ist und alle nachfolgenden Linsen einen Durchbruch für die Strahlung der vorhergehenden Linse bzw. Linsen aufweisen.

Die mit dem Detektor **37** detektierten Interferenzsignale gehen als elektrische Signale auf eine Auswerteelektronik **45**. Je nach erreichbarer elektrischer Signalstärke und erreichbarem Signal/Rauschverhältnis ist in dieser Auswerteelektronik **45** ein mehr oder weniger hoher Aufwand zu treiben. In der Regel hat die Auswerteelektronik **45** einen Vorverstärker **V,** ein Signalfilter **F,** einen Gleichrichter **GR** und ein Tiefpassfilter **TPF.** Vorzugsweise werden die elektrisch bearbeiteten analogen Signale in digitale Signale zu einer weiteren Verarbeitung bzw. Speicherung umgewandelt. Auch können die digitalisierten Signale über Netzwerke [**L**ocal **A**rea **N**etwork **LAN** (z.B. Ethernet) oder **W**ide **A**rea **N**etwork **WAN** (z.B. Internet)] mit anderen Daten verglichen bzw. zur Auswertung versandt werden. Auch könnten die ermittelten Daten auf einem Monitor **M** in entsprechender Form dargestellt werden.

Die Reflektoren können wie in **Figur 4** dargestellt als n Elemente **31a, 31b,** .... nebeneinander mit einem gegenseitigen Versatz $e_2$ analog zum Versatz $d_2$ in Richtung zur Referenzstrahleinfallsrichtung angeordnet werden. Die Reflektoren können jedoch auch wie in **Figur 5** angedeutet hintereinander angeordnet werden. Um die Darstellung nicht zu überlasten und in Analogie zur **Figur 4** zeigt auch **Figur 5** nur zwei Reflektoren **49** und **50.** In Analogie zur Darstellung in **Figur 4** ist auch hier eine Kollimatorlinse 51 vorhanden, welche die aus einem Faserende **53** austretende Freiraumreferenzstrahlung kollimiert. Eine rotierende Blende **35,** wie in **Figur 4** verwendet, wird hier nicht benötigt. Die kollimierte Freiraumreferenzstrahlung **54** trifft nun auf einen ersten teilreflektierenden Reflektor **50** und danach auf einen 100% reflektierenden Reflektor **49**. Beide Reflektoren **49** und **50** sind in einem zum Abstand $d_2$ analogen Abstand $e_2$ angeordnet. Die Teilreflexion des Reflektors **50** wählt man dann entsprechend der Reflexion der Messbereiche. Beide Reflektoren **49** und **50** sind auch hier auf einer gemeinsamen Basis 55 angeordnet. Die Basis **55** führt hier analog zur Basis **39** die periodische Oszillation für die Laufzeitänderung durch (gekennzeichnet durch einen Doppelpfeil 56). Eine Messlängenadaptation kann dann durch eine relative Verschiebung der beiden Reflektoren **49** und **50** gegeneinander erreicht werden. Sollen mehrere Bereiche ausgemessen bzw. zueinander in Bezug gebracht werden, werden mehrere Reflektoren verwendet, wobei immer der Hinterste ein 100% Spiegel sein sollte. Die Teilreflexionen der davor stehenden Reflektoren sind aufeinander bzw. auf die Reflexion des Messbereichs anzupassen.

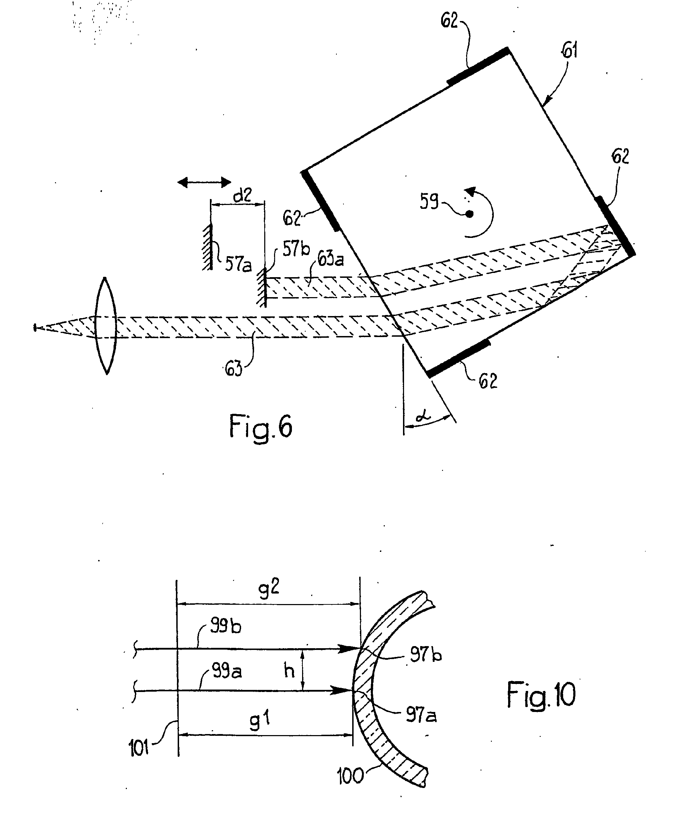

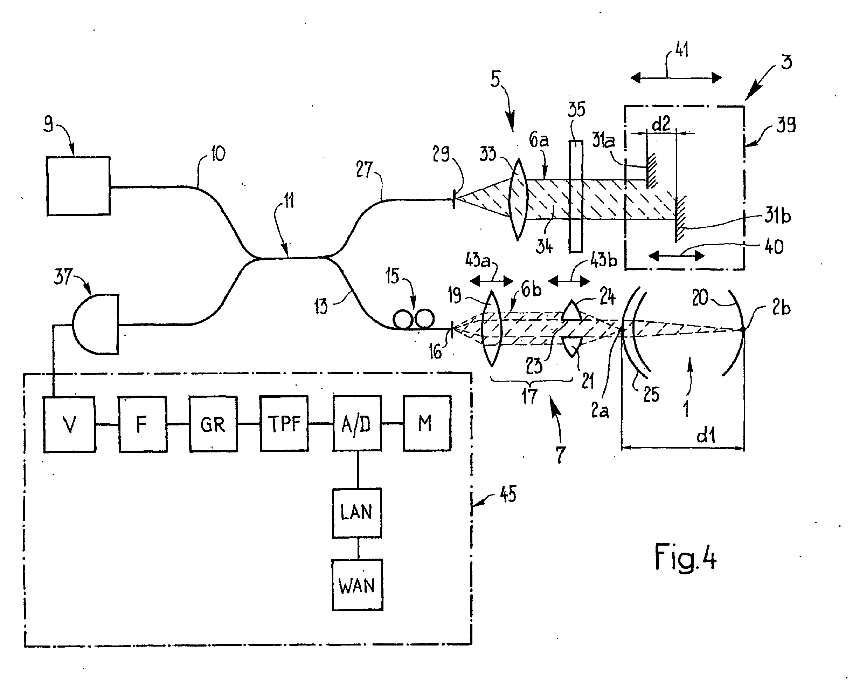

Neben einer Reflektoranordnung, wie sie in den **Figuren 4** und **5** dargestellt ist, zeigt **Figur 6** eine weitere beispielsweise Anordnung. Bei der in **Figur 6** gezeigten Anordnung sind die Reflektoren, hier mit **57a** und **57b** bezeichnet, im Gegensatz zu den oben erläuterten Ausführungen während des Messvorgangs stillstehend. Eine Verstellung der Reflektoren **57a** und **57b** wird nur dann vorgenommen, wenn sich der Messaufbau ändert. Den Reflektoren **57a** und **57b** ist ein um seine Mittelachse **59** rotierender transparenter Würfel **61** als sogenanntes Wegvariationselement vorgeschaltet. Ein derartiges Wegvariationselement **61** ist in der WO 96/35100 beschrieben. Die Aussenseiten des Würfels weisen verspiegelte Teilflächen **62** auf, an denen der in den Würfel **61** eindringende kollimierte Referenzfreiraumstrahl **63** mit einem Strahlengang, wie in **Figur 6** gezeigt, reflektiert wird. In Folge der Würfelrotation ergibt sich eine Bewegung des aus dem Würfel austretenden Strahles **63a** senkrecht zur Oberfläche der Reflektoren **57a** und **57b,** d.h. der austretende Strahl wandert zwischen den beiden Reflektoren **57a** und **57b** Spiegeln hin und her. Eine Bestrahlung der versetzten Reflektoren **57a** und **57b** zeitlich nacheinander ist zwar auch mit der in **Figur 4** beschriebenen rotierenden Blende **35** möglich, jedoch ergibt sich hier eine grosse Strahlungsabschwächung im Referenzstrahl. Diese Strahlungsabschwächung wird vollständig bei der Anordnung mit dem rotierenden Würfel **61** eliminiert.

Anstelle der beiden Reflektoren **57a** ud **57b** kann auch ein nicht dargestellter transparenter Quader mit zwei gegenüberliegenden zueinander parallelen Wänden verwendet werden. Die dem rotierenden Würfel **61** zugewandte Quaderseite wird teilweise reflektierend und teilweise transmittierend ausgebildet und die abgewandte Quaderseite totalreflektierend. Der Abstand der beiden Quaderflächen beträgt $d_2$. Vorzugsweise wird man den Quader aus Glas ausbilden. Er kann fest montiert sowie auch auf einem Translatortisch angeordnet sein, um eine Anpassung an unterschiedliche Messvorgänge vornehmen zu können. Bei Messungen am menschlichen Auge wird $d_2$ entsprechend der Augenlänge gewählt.

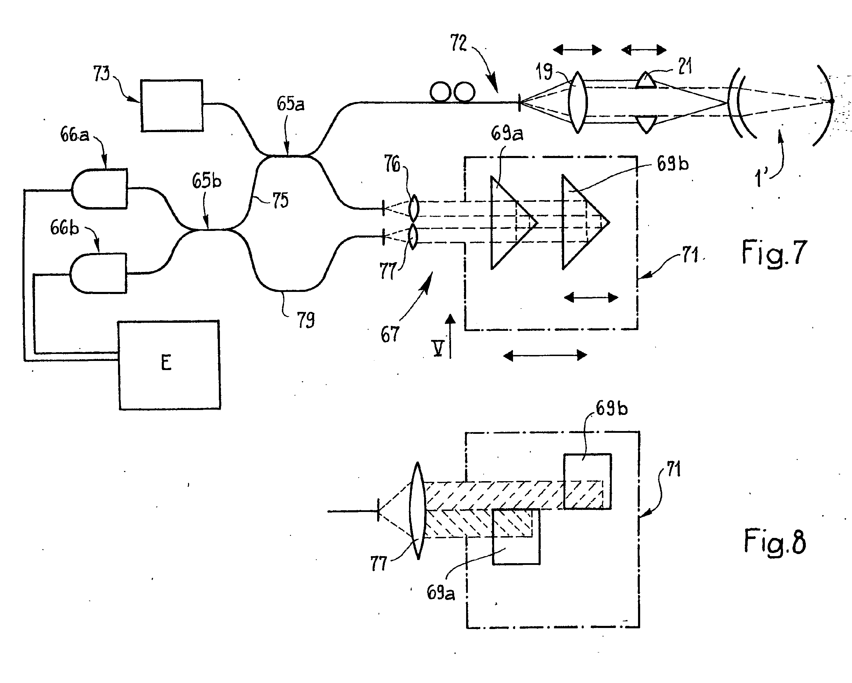

Eine Ausführungsvariante zu der in **Figur 4** dargestellten optischen Anordnung zeigt **Figur 7.** Im Gegensatz zu der in **Figur 4** dargestellten Anordnung sind hier zwei Faserkoppler **65a** und **65b** und zwei Detektoren **66a** und **66b** vorhanden. Auch weist jetzt der Referenzarm **67** anstelle der in **Figur 4** verwendeten planen Reflektoren **31a** und **31b** zwei als Retroreflektoren wirkende Prismen **69a** und **69b** auf, welche auch hier auf einer oszillierend bewegbaren Basis **71** angeordnet sind. Die beiden Prismen **69a** und **69b** sind zur Erzeugung des Laufzeitunterschieds hintereinander und nebeneinander, wie die Seitenansicht in **Figur 8** zeigt, versetzt. Die in der Seitenansicht gezeigte seitliche Versetzung ist notwendig, sonst würde das Prisma **69a** das Prisma **69b** abdekken. Der Messarm **72** ist analog zum Messarm **7** in **Figur 4** ausgebildet.

Die in **Figur 7** von einer analog zur Strahlungsquelle **9** ausgebildeten Strahlungsquelle **73** ausgehende kurzkohärente Strahlung wird im Faserkoppler **65a** in den Messarm **72** und in den Referenzarm **67** aufgeteilt. Die von den im Gegenstand, hier mit **1'** gekennzeichnet, auszumessenden Bereichen reflektierte Strahlung wird nach dem Faserkoppler **65a** zum Faserkoppler **65b** über eine Faser **75** geführt. Die von den Prismen **69a** und **69b** reflektierte d.h. umgeleitete, mit der Linse **76** kollimierte Referenzfreiraumstrahlung tritt über eine Fokussierlinse **77** in eine zum Faserkoppler **65b** führende Faser **79** ein. Im Faserkoppler **65b** erfolgt dann die interferierende Überlagerung der Strahlung aus dem Messarm **72** mit derjenigen aus dem Referenzarm **67**. Die Detektion erfolgt mit den beiden Detektoren **66a** und **66b**. Durch die Verwendung von zwei Detektoren **66a** und **66b** kann das Signal/Rauschverhältnis und somit die Messempfindlichkeit deutlich verbessert werden.

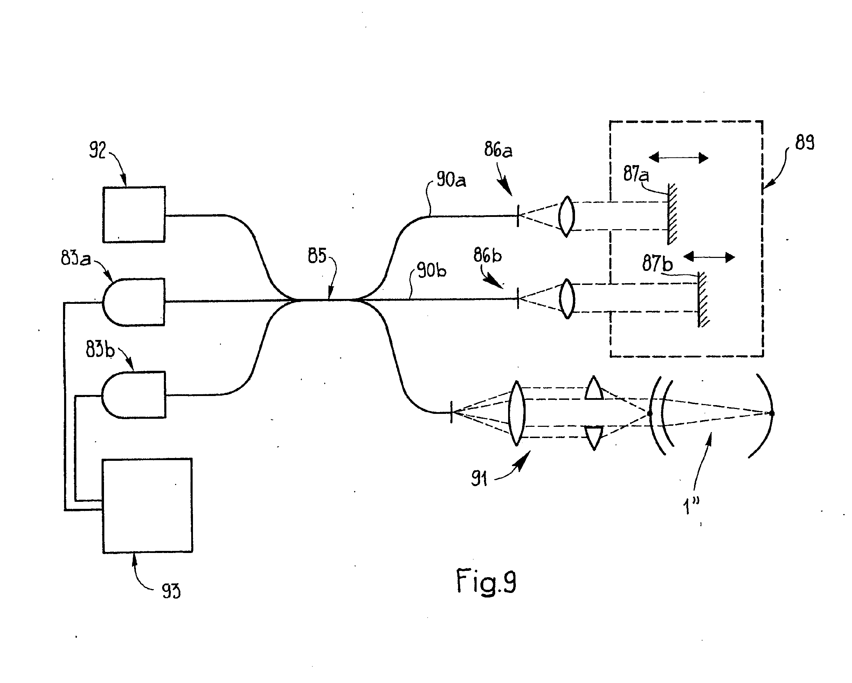

**Figur 9** zeigt eine weitere Variante zu den in den **Figuren 4** und **7** dargestellten Messanordnungen. Analog zur Darstellung in **Figur 7** wird auch hier mit zwei Detektoren **83a** und **83b** gearbeitet. Anstelle der 2 x 2 Faserkoppler **11** bzw. **65a** und **65b** in den **Figuren 4** und **7** wird hier jedoch ein 3 x 3 Faserkoppler **85** verwendet. Auch gibt es jetzt zwei Referenzarme **86a** und **86b,** in die jeweils ein- und dieselbe Strahlung nach einer Laufzeitänderung durch den jeweiligen Reflektor **87a** und **87b** rückreflektiert wird. Auch die Reflektoren **87a** und **87b** sind gegeneinander verstellbar und auf einer oszillierenden Basis **89** angeordnet. Die rückreflektierte Strahlung jedes Reflektors **87a** und **87b** wird in dieselbe Faser **90a** bzw. **90b** eingekoppelt, aus dem sie gekommen ist. Die von einer Strahlungsquelle **92** kommende kurzkohärente Strahlung wird durch den Faserkoppler **85** in den Messarm **91** und die beiden Referenzarme **86a** und **86b** aufgeteilt. Die im Messarm **91** von den Bereichen im Gegenstand **1"** reflektierte Messstrahlung sowie die beiden reflektierten Strahlungsanteile aus den Referenzarmen **86a** und **86b** werden im Faserkoppler **85** interferierend überlagert und dann von den beiden Detektoren **83a** und **83b** detektiert und mit der an sie angeschlossenen Auswertelektronik **93** ausgewertet.

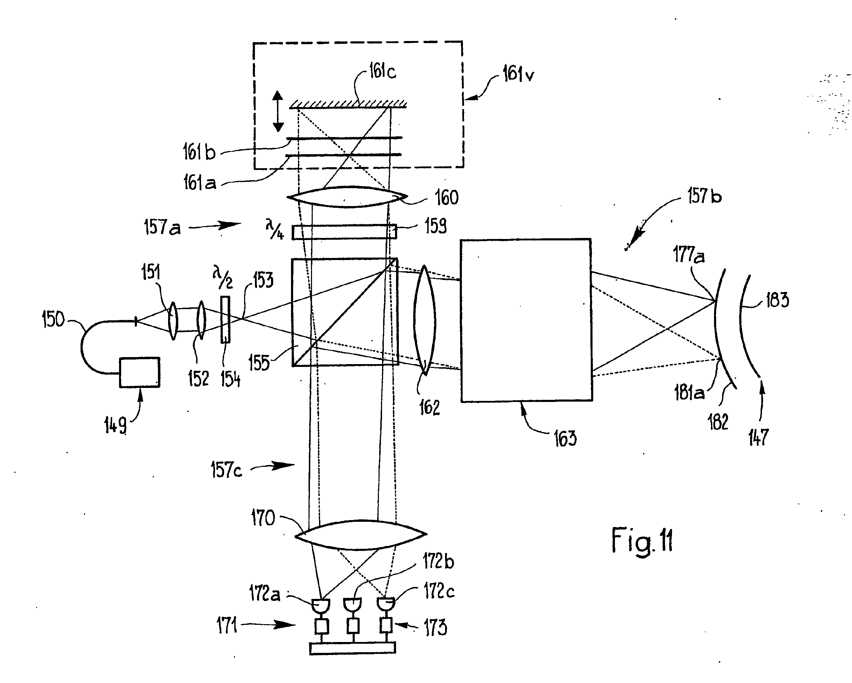

In den vorgängig beschriebenen **Figuren 4** bis **9** werden Messungen ausgeführt, bei denen eine Dicke ermittelt wird. Hierzu wird der erste Messstrahl auf einen ersten Bereich (Punkt) fokussiert und der zweite Messstrahl auf einen zweiten, hinter dem ersten Bereich liegenden Bereich (Punkt) fokussiert. Erster und zweiter Bereich lagen bisher auf einer optischen Achse. Man kann nun die erfindungsgemässe Vorrichtung auch derart aus- und umbilden, dass die Fokuspunkte der beiden Messstrahlen nebeneinander liegen. Werden die Messstrahlen seitlich nebeneinander gelegt, so kann bei einer Oberfläche, welche wenigstens einen Mindestreflexionsfaktor von $10^{-4}$ % hat, ein Oberflächenprofil bestimmt werden. Es wird hierzu, wie in **Figur 10** angedeutet ist, der Abstand $g_1$ eines ersten reflektierenden Ortes **97a** des ersten Messstrahls **99a** auf der Oberfläche **100** von einem Referenzpunkt bzw. einer Referenzebene **101** und der Abstand $g_2$ des zweiten reflektierenden Ortes **97b** des zweiten Messstrahls **99b** von der Referenzebene **101** ermittelt. Beide Messwerte werden in einer Speichereinheit in einer elektrischen Auswerteeinheit abgespeichert. Die Abstandsdifferenz $g_1$ und $g_2$ der beiden Messstrahlen **99a** und **99b** von der Referenzebene **101** in Relation zu deren gegenseitigem Abstand h ergibt dann zwei Oberflächenkoordinaten. Aus diesen beiden Koordinaten kann dann auf den Oberflächenverlauf mit Näherungsverfahren geschlossen werden, sofern die Art der Oberfläche bekannt ist. Die Art der Oberfläche ist beim menschlichen Auge bekannt. Werden mehrere Messstrahlen verwendet oder mehrere Messungen mit seitlich versetzten Messstrahlen vorgenommen, kann die Oberfläche genauer ermittelt werden.

In der Ophthalmologie spielt bei der Anpassung von intraokulären Linsen in der Katarakt-Behandlung nicht nur die Augenlänge und die Vorderkammertiefe eine wesentliche Rolle, sondern auch das Krümmungsprofil der Hornhaut, vor allem in deren Zentrum. Alle diese Werte können mit der erfindungsgemässen Vorrichtung bestimmt werden.

Zur Ermittlung des Profils genügen im Minimalfall zwei bestimmte Krümmungsradien der zentralen Hornhaut, nämlich ein Krümmungsradius in horizontaler und einer in vertikaler Richtung. Sind diese beiden Radien verschieden, spricht man von einem (zentralen) Astigmatismus. Die Krümmungsradien können mit Hilfe bekannter geometrischer Algorithmen ermittelt werden, wenn pro zu bestimmenden Kreisbogen, wie bereits oben ausgeführt, ausgehend von einer Referenzebene (hier **101**) der Abstand unter einem vorgegebenen Winkel (hier der normale Abstand $g_1$ und $g_2$) und der Abstand (hier **h**) der Kurvenpunkte (hier **97a** und **97b**) voneinander bekannt sind. Die Abstände $g_1$ und $g_2$ sind aus dem augenblicklichen Ort des bzw. der Reflektoren bzw. des augenblicklichen Drehwinkels der Weglängenvariationseinheit (rotierender Würfel) bei auftretender Interfenzerscheinung ermittelbar. Als Referenzwert wird eine vorgegebene Lage der Reflektoren bzw. der Wegvariationseinheit verwendet. Wird eine Weglängenvariationseinheit mit einem rotierenden Würfel (beispielsweise wie in der WO 96/35100 beschrieben) verwendet, wird man bevorzugt dessen Nullgrad-Position als Referenz verwenden, bei der der einfallende Strahl senkrecht auf die erste Würfeloberfläche trifft. Anstelle vom minimal drei Messstrahlen zur Ermittlung der beiden zentralen Krümmungsradien können auch mehrere Messstrahlen verwendet werden, um eine exaktere Krümmungsradienausmessung vorzunehmen. Auch kann gleichzeitig Dicken- und Radienmessung vorgenommen werden, wie unten ausgeführt wird.

Die in **Figur 11** anhand eines optischen Blockschaltbildes beschriebene Vorrichtung dient zur Ermittlung eines Oberflächenprofils sowie diverser Dicken bei einem transparenten bzw. diffusiven Gegenstand, hier einem menschlichen Auge **147.** Der in **Figur 11** schematisch dargestellte optische Aufbau ähnelt in weiten Teilen demjenigen der **Figur 4,** die Fasern sind hier als Variante durch Freiraumstrahlungen ersetzt. Auch hier ist eine Strahlungsquelle **149** vorhanden, welche beispielsweise eine superlumineszente Diode sein kann. Die Strahlung der Strahlungsquelle **149** wird hier über eine Faser **150** geführt, wodurch sich eine Ortsunabhängigkeit von der Strahlungsquelle 149 sowie der Mess- und Auswerteeinheit erreichen lässt. Die aus der Faser **150** austretende Strahlung wird mit einer Linse **151** kollimiert und mit einer zweiten, nachfolgenden Linse **152** fokussiert. Zwischen dem Fokuspunkt **153** und der Linse **152** ist zur "Drehung" der Polariationsrichtung der Strahlung eine λ/2-Platte **154** angeordnet. Anschliessend folgt ein Strahlteiler **155,** mit dem die Strahlung in den Messarm **157b** und den Referenzarm **157a** aufgeteilt wird. Der Referenzarm **157a** hat auf den Strahlteiler **155** folgend eine λ/4-Platte **159,** gefolgt von einer Linse **160,** mit der die vom Strahlteiler **155** kommende Strahlung kollimiert wird. Auf die Linse **160** folgt ein erster und ein zweiter teildurchlässiger Reflektor **161a** und **161b** sowie ein 100% Reflektor **161c.** Alle drei Reflektoren **161a, 161b** und **161c** sind entsprechend der durchzuführenden Messung gegeneinander verstellbar und auf einer oszillierenden Basis **161v** für die Laufzeitänderung angeordnet.

Im Messarm **157b** folgt auf den Strahlteiler **155** eine Kollimationslinse **162** und ein Linsensystem **163** analog der Linse **21** in **Figur 4.**

Die vom Auge **147** rückreflektierte Strahlung wird mit der vom Referenzarm **157a** kommenden Referenzstrahlung überlagert und im Detektorarm **157c** über eine Linse **170** auf ein Detektorarray **171** geführt, wobei der Einfachheit halber nur eine lineare und keine zweidimensionale Darstellung von lediglich drei nebeneinander angeordneten Detektoren **172a, 172b und 172c** vorgenommen worden ist. Jedem Detektor **172a, 172b** und **172c** ist eine elektronische Schaltung **173** beispielsweise mit einem Verstärker, Dopplerfrequenzfilter, Gleichrichter und Tiefpassfilter nachgeschaltet. Die detektierten Messsignale werden dann über einen Analog-Digital-Wandler und einen Computer mit der Speichereinheit verarbeitet und auf einem Bildschirm dargestellt.

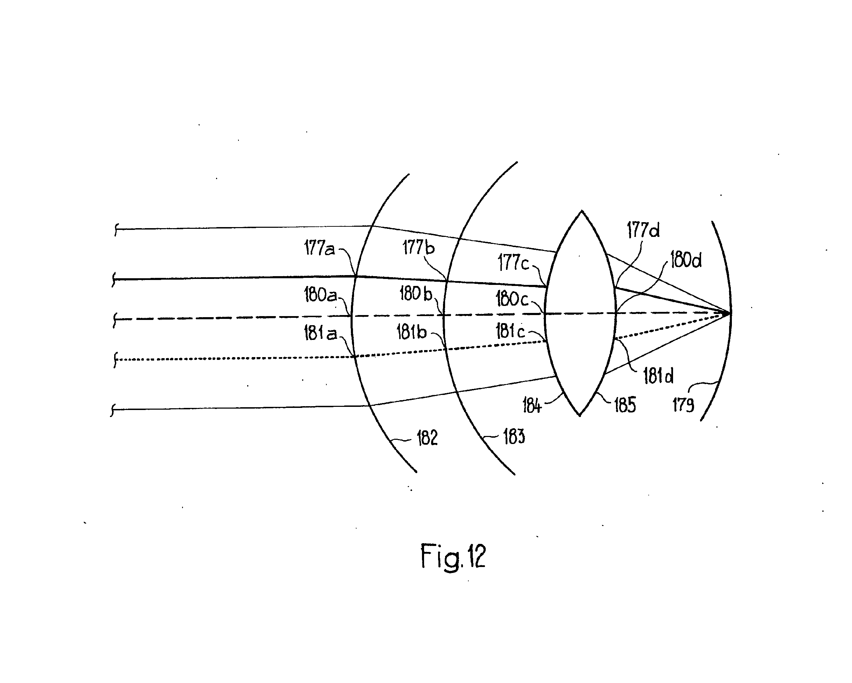

Mit der in **Figur 11** schematisch dargestellten Vorrichtung kann die Augenlänge, die Corneadicke, die Vorderkammertiefe, die Linsendicke, die Glaskörpertiefe und die Retinaschichtdicke gleichzeitig an verschiedenen Stellen gemessen werden. Da an verschiedenen Stellen gemessen werden kann, können auch Oberflächenprofile rechnerisch ermittelt werden. Um dies zu verdeutlichen, sind in **Figur 12** drei seitlich versetzte Strahlengänge ausgezogen, gestrichelt und punktiert dargestellt, welche auf die Detektoren **172a, 172b** und **172c** geführt werden. Der durchgezogene Strahl kommt, wie der besseren Übersichtlichkeit wegen vergrössert in **Figur 12** dargestellt, von den Orten **177a, 177b, 177c, 177d** und der Retina **179.** Mit Hilfe eines Detektorarrays, bestehend aus m x n Photodetektoren, ist es möglich m x n Orte auf oder im Auge **147** z.B. auf der Corneavorderfläche **182,** der Corneahinterfläche **183,** der Augenlinsenvorder- bzw. hinterfläche **184** und **185** gleichzeitig zu messen und auszuwerten. Nach einer gewissen Zeitdauer, welche abhängig ist von der Bewegungsgeschwindigkeit der Basis **161v,** werden dann die mit **"b",** dann mit **"c"** und mit **"d"** gekennzeichneten Orte detektiert und ausgewertet (siehe Figuren 11 und 12).

Die Linsen **160** und **162** können je nach Verwendung als ein- oder zweidimensionales Linsenarray ausgebildet sein.

Zum besseren Verständnis des Messvorganges sind in **Figur 11** zusätzlich die "Strahlenbegrenzungen" zum und vom Ort **177a** ausgezogen sowie zum und vom Ort 181a punktiert im Referenz-, Mess- und Detektorarm **157a, 157b** und **157c** dargestellt. Die ausgezogenen bzw. punktierten Linien zeigen die zwei Randstrahlen im Referenz-, Mess- und Detektorarm **157a, 157b** und **157c,** welche die Messung der Raumkoordinaten des Ortes **177a** bzw. **181a** ermöglichen, d.h. mit diesen Strahlen interferieren.

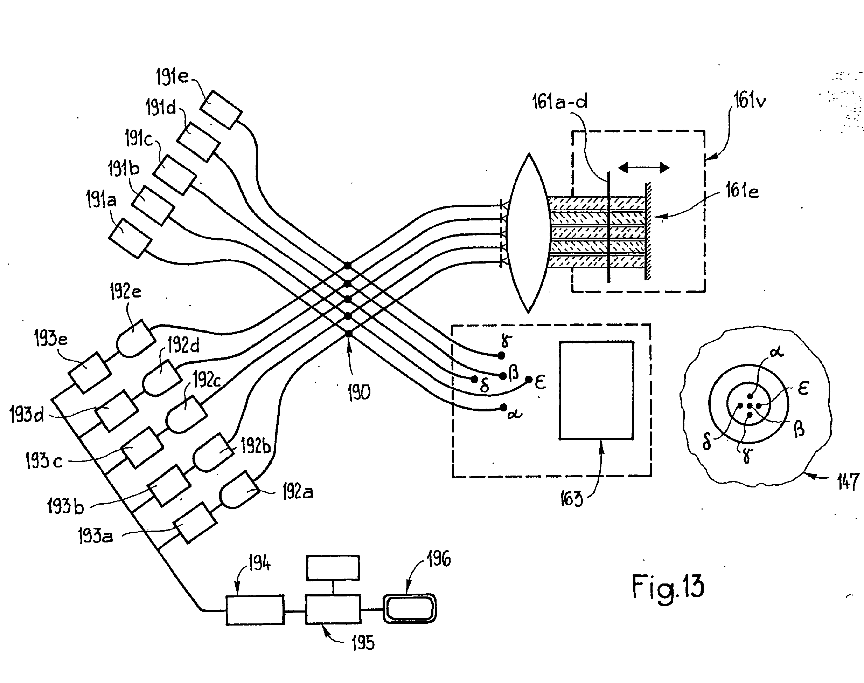

In **Figur 13** ist eine als faseroptisch paralleles kurzkohärentes Reflektormeter zu bezeichnende Vorrichtung skizziert. Diese Ausführungsvariante erlaubt beispielsweise die simultane Messung von vier zentralen Krümmungsradien der Hornhautvorderfläche in horizontaler (links und rechts) und vertikaler (oben und unten) Richtung. Zusätzlich ist auch die simultane Messung von vier zentralen Krümmungsradien der Hornhautrückseite möglich. Diese Vorrichtung hat fünf 2 x 2 Singelmodefaserkoppler **190,** fünf Strahlungsquellen **191a** bis **191e,** fünf Detektoren **192a** bis **192e** mit zugehörender Schaltung **193a** bis **193e,** Analog-Digital-Wandler **194,** Computer **195** und Anzeige **196.** Die restlichen Elemente und Einheiten (insbesondere **161a** bis **161e** und **163),** entsprechen denjenigen der **Figur 11.**

Anstatt eine Basis mit der Reflektoranordnung **161a** bis **161e** oszillierend aufzubauen, kann auch nach der Kollimatorlinse der oben beschriebene rotierende Würfel mit einer Stufenspiegelanordnung analog zu **Figur 6** verwendet werden.

Die Lage der reflektierenden Elemente **31a/b, 49/50, 57a/b, 69a/b, 87a/b** sowie **161** wird jeweils für das auszumessende Objekt (hier in der Regel das Auge, wobei auch andere Objekte ausmessbar sind) eingestellt. Zum Auffinden der optimalen Lage der reflektierenden Elemente im Referenzarm können diese Elemente auf einem nicht dargestellten Translatortisch angeordnet werden. Mit diesem Tisch verschiebt man nun die reflektierenden Elemente schrittweise (z.B. in Schritten von 0,1 mm bis 1 mm). Nach Ausführung jeweils eines Schrittes stoppt der Translatortisch zur Durchführung einer Messung: Durch periodisches Scannen einer vorgegebenen Tiefe mittels des Weglängenvariators (z.B. des Weglängenvariators **41, 55, 61, 71, 89,** ...) wird nach Reflexionssignalen gesucht. Ist bei diesem "Tiefenscann" kein Reflexionssignal gefunden worden, erfolgt der nächste Verfahrensschritt des Translatortisches. Dieses Verfahren wird so-lange wiederholt bis entsprechende Reflexionen vorhanden sind. Dieser Suchvorgang kann selbstverständlich auch automatisch durch eine Steuereinrichtung erfolgen.

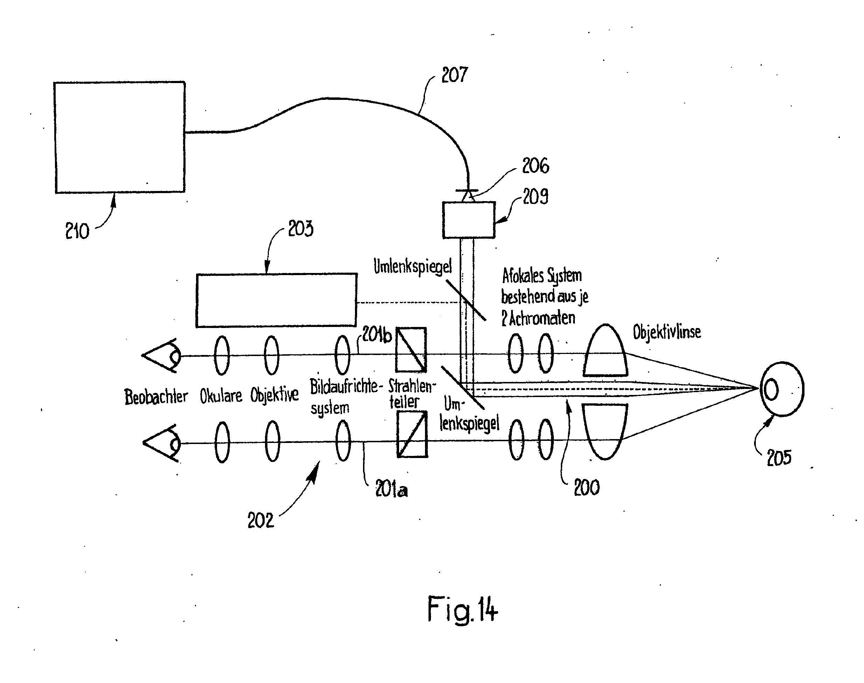

Die oben beschriebene erfindungsgemässe Vorrichtung kann nebst ihren Ausführungsvarianten zusammen mit bereits bestehenden Geräten verwendet werden. Diese Vorrichtung kann man z.B. in ein Spaltlampengerät für die Augenuntersuchung einbauen oder mit diesem kombinieren. Es kann dann der Messstrahl als Freiraumstrahl entweder via Strahlteiler in den Beleuchtungsstrahlengang, beim Mikroskop ebenfalls via Strahlteiler in einen Beobachtungsstrahlengang oder beim Mikroskopobjektiv oder mit einem Umlenkspiegel **199** in einen Mittelkanal **200** eines Stereomikroskops **202** eines Spaltiampengeräts, wie in **Figur 14** gezeigt, eingekoppelt werden. Der Mittelkanal **200** liegt zwischen den beiden Strahlengängen **201** a und **201** b des Stereomikroskops **202.** In **Figur 14** ist zudem noch eine Fixationslichtquelle **203** gezeigt. Durch eine Betrachtung der Fixationsquelle **203** richtet der Patient sein Auge **205** auf einen vorgegebenen Ort aus und hält es meistens auch auf diesem unbeweglich fixiert. Der Messstrahl **206** tritt (analog einer Vorrichtungsanordnung wie sie die **Figuren 4, 7** und **9** zeigen) aus einer Faser **207** aus und durchläuft ein analog zum Linsensystem **17** ausgebildetes Linsensystem **209** mit einem optionalen transversalen Scanner. Die restlichen Elemente der erfindungsgemässen Vorrichtung sind in einem kompakten Basisgerät **210** eingebaut.

Durch die Verstellung des Spaltlampengeräts in den drei räumlichen Koordinaten, bevorzugt mit einem sogenannten Lenkhebel, wird dann auch der Messstrahl entsprechend verstellt. Anstatt das ganze Spaltlampengerät zusammen mit dem Messstrahl zu verstellen, können auch beide unabhängig voneinander verstellt werden. Wie bereits oben angedeutet, verwendet man bei einer Verstellung nur des Messstrahls bevorzugt eine "faseroptische" Ausführung analog zur Darstellung in **Figur 4.**

Bei einer Kombination mit einem mit Placidoringen ausgerüsteten Videokeratographen erfolgt eine Einkopplung des Messstrahls in Richtung der Beleuchtungsachse des Videokeratographen mit Hilfe eines kleinen Strahlteilers.

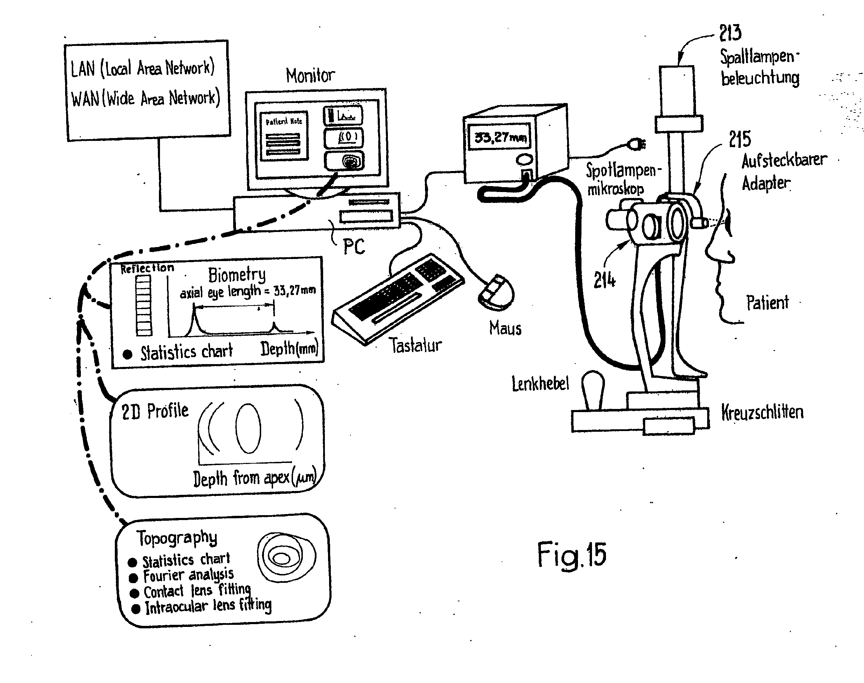

Anstatt den Messstrahlengang, wie oben beschrieben, in ein Stereomikroskop zu integrieren, kann er auch bei einem Spaltlampengerät **213** über einen auf das Mikroskop **214** aufsteckbaren Adapter **215** zugeführt werden, wie in **Figur 15** skizziert ist.

Bei den oben beschriebenen Ausführungsvarianten gilt allgemein, dass sämtliche Strahlteiler, ob sie nun Faserkoppler oder Strahlteilerwürfel sind, als polarisierende Strahlteiler ausgebildet sind. Auch strahlen die Strahlungsquellen **9, 73,149** und **191a** bis **191e** eine polarisierte Strahlung in ihrem Quellenstrahl aus. Immer wenn eine Interferenz detektierbar ist, sind die Längen der optischen Wege im Referenz- und im Messarm gleich lang, wobei die optische Weglänge im Referenzarm im Hertzbereich veränderbar ist. Das die Strahlung im Messarm auf die betreffenden Bereiche fokussierende Linsensystem z.B. **17** kann bei gewissen Anwendungen weggelassen werden. Es kann z.B. für die Augenlängenmessung die Fokussierung des Messstrahls von der Brechkraft des Auges übernommen werden.

Der optische Laufzeitunterschied bzw. die optischen Laufzeitunterschiede der im Referenzarm angeordneten Reflektoren werden immer so eingestellt, wie sie einem zu erwartenden ungefähren Messergebnis entsprechen würden. D.h. durch die Messung wird somit immer nur die Abweichung von einem zu erwartenden Messergebnis ermittelt. Da diese Abweichungen immer bedeutend kleiner sind, als wenn der gesamte Weg (Abstand, Dicke, ...) ausgemessen werden muss, kann mit einer bedeutend kleineren und damit rascheren Weglängenvariation (Laufzeitänderung) im Referenzarm gearbeitet werden. D.h. zeitlich gesehen, fallen die beiden Interferenzen sehr schnell hintereinander an; sie können sogar gleichzeitig erfolgen. Da bei Abständen, Dickenmessungen, ... beim Stand der Technik immer zwei zeitlich gestaffelte Messungen vorzunehmen waren, liegt nun bei der Erfindung das Messergebnis derart schnell vor, dass Ortsverschiebungen des auszumessenden Gegenstands die Messgenauigkeit nur unwesentlich beeinflussen.

Der gerade geschilderte Vorteil ist bei einer Augenlängenmessung an den Augen von Kindern, welche in der Regel nur schwer still zu stellen sind, von grossem Nutzen.

Ist eine Zuordnung der auftretenden Interferenzen zu den betreffenden reflektierenden Flächen gewünscht, so können anstelle eines einzigen Photodetektors zwei, jeder für eine Polarisationsrichtung, verwendet werden. Es wird dann die Strahlung der einen Polarisationsrichtung mittels eines polarisierenden Strahlteilers auf den einen Photodetektor und die Strahlung der anderen Polarisationsrichtung auf den anderen Photodetektor geleitet.

Die Strahlungsreflexion kann nun an bzw. in einem der Bereiche unterschiedlich hoch sein; auch können Reflexionen von Bereichen innerhalb eines Gegenstands, deren Abstand man bestimmen möchte oder sofern es sich um Schichten handelt, deren Dicke man ermitteln möchte, unterschiedlich sein. Um nun die reflektierte Intensität in einem gewissen Rahmen anpassen zu können, können $\lambda/2$- bzw. $\lambda/4$ Platten im Quellenstrahl und im Referenzstrahl angeordnet werden. Man kann nun die jeweilige Platte derart verstellen, dass in dem Strahl, dessen Strahlung schwach reflektiert wird, mehr Intensität eingekoppelt wird.

Die Weglängenänderung im Referenzarm beaufschlagt die Strahlungsfrequenz des Referenzstrahls mit einer Dopplerfrequenz $f_{Doppler}$ gemäss der Beziehung

$$f_{Doppler} = \frac{2 \cdot f_0 \cdot v_{scan}}{c} \, ,$$

wobei $f_0$ die Strahlungsfrequenz der Strahlungsquelle, $v_{scan}$ die Weglängenänderungsgeschwindigkeit und $c$ die Lichtgeschwindigkeit ist. (Mit der in der WO96/35100 beschriebenen Weglängenvariationseinheit ist die Dopplerfrequenz $f_{Doppler}$ annähernd konstant.) Diese Dopplerfrequenz weist auch das mit dem Photodetektor detektierte Interferenzsignal auf. Das vom Detektor erhaltene elektrische Signal kann somit mit einem elektronischen Bandpassfilter von der restlichen detektierten Strahlung getrennt werden. Hierdurch verbessert sich das Signal/Rauschverhältnis erheblich.

Die Eichung der oben beschriebenen Vorrichtungen kann dadurch erfolgen, dass in den Referenzarm die Strahlung einer hochkohärenten Strahlungsquelle (z.B. ein distributed feedback Laser) mit einem (nicht dargestellten) Strahlteiler eingekoppelt wird. Die eingekoppelte Strahlung interferiert dann mit einem Strahlungsteil, der an einem beliebigen Ort zwischen diesem Strahlteiler und dem Weglängenvariator an einem feststehenden Reflektor reflektiert wird. Die Kohärenz der hochkohärenten Strahlungsquelle ist grösser als die Wegvariationslänge des Variators. Über den Detektoren (oder auf einem separat hierfür eingerichteten Detektor) läuft dann ein Interferenzstreifenmuster. Der Abstand jeweils zweier Interferenzstreifen entspricht dann jeweils einer halben Wellenlänge. Durch ein (automatisches) Auszählen dieser Streifen ist eine Wegeichung des Weglängenvariators möglich. Da die hochkohärente Strahlung nicht auf das Auge des Patienten gelangen kann, kann deren Strahlungsleistung relativ hoch sein, so dass diese Detektion unkritisch ist. Die Wellenlänge der hochkohärenten Strahlung kann (muss es aber nicht) die gleiche Wellenlänge wie die für die Augenmessung verwendete kurzkohärente Strahlung haben.

Die mit den oben beschriebenen erfindungsgemässen Vorrichtungen ermittelten Dicken der Hornhaut können vorzugsweise in eine Beratung von Patienten einfliessen, bei denen eine refraktive Chirurgie mit LASIK (Laser-assisted in situ Keratomileusis) vorgenommen werden soll, in dem eine individuelle Berechnung eines Unterschieds zur kritischen Hornhautdicke mit Blick auf die aktuelle Hornhautdicke erfolgt. Hierzu wird man vorzugsweise die nachfolgenden neuen

**EP 1 494 575 B1**

Schritte vornehmen:

1. Es wird eine präoperative zentrale Hornhautdicke $d_z$ mit einer der Vorrichtungen ermittelt.

2. Von der ermittelten Hornhautdicke $d_z$ wird die bei LASIK übliche mittlere Flapdikke $d_f$ von typischerweise 160 $\mu$m (einstellbar) abgezogen.

3. Es wird ein (maximal möglicher) Pupillendurchmesser ermittelt, während dem das Auge typischen nächtlichen Lichtintensitätsbedingungen ausgesetzt ist. Der "nächtliche Pupillendurchmesser" kann durch Abdunkeln des Untersuchungsraums mit einer an die erfindungsgemässen Vorrichtungen bzw. deren Ausführungsvarianten angeschlossenen TV-Kamera ausgemessen werden. Eine derartige Kamera kann z.B. im Detektorarm via Strahlteiler mit einem entsprechenden Linsensystem angedockt werden. Die Messung des Pupillendurchmessers ist optional. Für eine Beratung können auch Standardwerte verwendet werden.

4. Es wird nun ein optimaler Ablationsdurchmesser S auf der Hornhaut festgelegt, der grösser sein muss als der nächtliche Pupillendurchmesser, um nach der Ablation Haloerscheinungen zu vermeiden.

5. Die mit LASIK zu erreichende Korrketur in Dioptrien ist aus vorgängigen Messungen (z.B. durch Kenntnis der Brechkraft einer bereits verwendeten Brille oder Kontaktlinse, welche der Patient bereits besitzt) bekannt.

6. Gemäss der Formel $t_0 = - (S^2 D)/3$ wird die für die gewünschte Korrektur notwendige zentrale Abtragungstiefe $t_0$ [in Mikrometer] für die gewünschte Korrektur berechnet, wobei S der optimale Ablationsdurchmesser in Millimeter und D die gewünschte Dioptrienänderung infolge der Ablation ist.

7. Es wird jetzt die zentrale stromale Restdicke $d_s = d_z - d_f - t_0$ berechnet, welche nach der LASIK-Operation erhalten werden würde.

8. Es wird abgeklärt, ob die Restdicke $d_s$ über einer kritischen zentralen stromalen Restdicke $d_k$ liegt. Eine mögliche Definition für die kritische zentrale stromale Restdicke $d_k$ ist beispielsweise $d_k = a \cdot d_z - b$, wobei a = 0,58 und b = 30 $\mu$m als Standardwerte angenommen werden.

9. Ist nun $d_s$ grösser als $d_k$ kann eine LASIK-Operation empfohlen werden.

Die oben angeführten Verarbeitungsschritte können selbstverständlich über einen Rechner automatisiert werden.

Das Vorgehen bei einer Hyperopiekorrektur erfolgt analog. Die Hornhautdicke muss dann jedoch peripher an der Stelle der maximalen Abtragung gemessen werden; die unter Punkt 6 angegebene Formel ist dann entsprechend zu ersetzen.

Den oben angeführten Dicken- und Profilmessungen am Auge kann eine Bestimmung dessen Brechkraftverteilung hinzugefügt werden. Um dies zu erreichen, wird die Linse **162** in **Figur 11** durch ein (nicht dargestelltes) Linsenarray mit p x q Linsen ersetzt. Hierdurch wird die von der Strahlungsquelle **149** kommende Strahlung in p x q getrennte (nicht dargestellte) Teilstrahlen auf das Auge abgebildet. Das Linsenarray ist auf das Auge zu- bzw. von diesem wegbewegbar. Es wird nun in eine derartige Position gebracht, dass eine Fokussierung wenigstens teilweise auf der Retina erfolgt. An einem Ort zwischen der Augenoberfläche und der Linse **170** wird nun ein weiterer Strahlteiler eingesetzt und die Retina mit einer TV-Kamera betrachtet. Falls nun die örtliche Verteilung der Lichtpunkte auf der Retina von der durch das Linsenarray erzeugten Punktverteilung abweicht, ist die Brechkraftverteilung oder die Abbildungseigenschaft des Auges nicht ideal, d.h. das Auge bildet eine auf die Cornea auftreffende ebene Wellenfront nicht optimal ab. Diese Abweichung (z.B. sphärische Aberration, Koma usw.) kann dann auf einem Monitor dargestellt werden.

Bekannte Tonometer (Augendruckmessgeräte) haben den Nachteil, dass sie den intraokularen Druck nur indirekt messen können. Die Messung erfolgt beispielsweise über eine Kraft, welche notwendig ist, um eine Hornhautoberfläche auf einer vorgegebenen Fläche abzuplatten (Applationstonometer). Die "abplattende" Kraft ist aber abhängig von der Hornhautdicke und der Krümmung der Hornhaut. Die bekannten Tonometer gehen von einer standardisierten Hornhautnormaldicke und -normalkrümmung aus. Bei einer Abweichung der Hornhaut von den Standardwerten stimmt dann ein derart ermittelter Augeninnendruck nicht mit dem tatsächlichen überein. Je dicker oder je stärker die Hornhaut gekrümmt ist, desto stärker weicht der bekanntermassen ermittelte Innendruck vom tatsächlichen nach oben ab. Das kann dazu führen, dass wegen vermeintlich zu hohem Augendruck Medikamente zur Augendrucksenkung verabreicht werden, welche nicht nötig bzw. sogar schädlich sind. Diese Fehlmessung bzw. Fehlinterpretation kann jedoch auch dazu führen, dass beispielsweise ein Glaukom erst verspätet diagnostiziert wird.

**EP 1 494 575 B1**

Er wird nun vorgeschlagen, die erfindungsgemässe Vorrichtung mit einem Tonometer zu kombinieren. Der mit einem bekannten Tonometer gemessene ("falsche") Augeninnendruck wird rechnerisch unter Verwendung der mit der erfindungsgemässen Vorrichtung ermittelten Hornhautkrümmung und der Hornhautdicke korrigiert. Die Korrektur kann durch eine Eingabe der Werte in einen Rechner erfolgen oder automatisch durch eine elektronische Verknüpfung der beiden Geräte.

Die erfindungsgemässen Vorrichtungen, ihre Ausführungsvarianten sowie ihre Messgeräte können vernetzt werden, wodurch auch eine Aufbereitung und Abspeicherung von Daten auch an entfernten Orten vorgenommen und mit anderen Daten verglichen werden kann.

Die erfindungsgemässe Vorrichtung dient wie bereits oben teilweise erwähnt zur ophthalmologischen Messung

- ➢ der Corneadicke, des Corneadickenprofils, des Corneavorder- sowie -rückseitenprofils;
- ➢ der Vorderkammertiefe, des Vorderkammertiefenprofils,
- ➢ der Linsendicke, des Linsendickenprofils, des Linsenvorder- sowie -rückseitenprofils,
- ➢ der Glaskörpertiefe, des Glaskörperprofils,
- ➢ der Retinaschichtdicke, des Retinaoberflächenprofils,
- ➢ der Epitheliumdicke, des Epitheliumprofils, des Epitheliumvorder- und -rückflächenprofils,
- ➢ der cornealen Flapdicke, des Flapdickenprofils, des Flapvorder- und -rückseitenprofils, der Flapposition,
- ➢ der cornealen Stromadicke, des Stromaprofils, des Stromavorder- und -rückflächenprofils.

Weitere Messungen können bei postoperativen Nachuntersuchungen nach refraktiver Chirurgie vorgenommen werden.

**Patentansprüche**

1. Modularer aufbaubarer ophthalmologischer Untersuchungs- und/oder Behandlungsplatz mit einem unmittelbar vor dem Patientenauge **(301)** positionierbaren Patientenmodul **(303),** mit einer vom Patientenmodul entfernt angeordneten Auswerteeinheit **(317),** mit einer ebenfalls vom Patientenmodul **(303)** entfernt angeordneten Beleuchtungseinheit **(305),** und mit wenigstens einem Faserkopplungsteil für eine ein entsprechendes Kupplungsgegenstück **(302)** aufweisende optische Faser **(304)** zur lösbaren Verbindung der vom Patientenmodul **(303)** örtlich entfernten Beleuchtungseinheit **(305),** wobei das Patientenmodul **(303)** einen mit dieser optischen Faser **(304)** zusammenwirkenden Kollimator **(310a)** hat, mit dem die Strahlung dieser Faser **(304)** in eine Freiraumstrahlung **(307)** umwandelbar ist, mit einer Beobachtungseinheit **(325a/b, 326a/b, 315; 322, 323),** welche im Patientmodul **(303)** angeordnet ist, mit einer, mit der Auswerteeinheit **(317)** signalmässig verbundenen, optischen Messanordnung **(312, 311, 309, 313)** mit wenigstens einer weiteren optischen Faser **(309)** und einem weiteren Kollimator **(310b),** mit dem die Strahlung der wenigstens einen weiteren Faser **(309)** in eine Freiraumstrahlung **(312)** umformbar ist, wobei der weitere Kollimator **(310b)** im Patientenmodul **(303)** angeordnet ist und das Patientenmodul **(303)** wenigstens einen weiteren Faserkopplungsteil **(311)** zur lösbaren Ankopplung der zur Messanordnung **(312, 311, 309, 313)** gehörenden, wenigstens einen weiteren Faser **(309)** hat, wobei die weitere Faser **(309)** ein dem wenigstens einen weiteren Faserkopplungsteil **(311)** entsprechendes weiteres Kopplungsgegenstück hat und die Messanordnung einen "Michelson-Interferometer"-artigen, eine kurzkohärente Strahlungsquelle **(9; 73; 92; 149; 191a-e)** aufweisenden, optischen, im Wesentlichen faseroptisch aufgebauten Anordnung hat, ein Messarm **(7; 72; 91; 157b)** der Messanordnung eine optische Faser und einen Kollimator hat, mit welchem Strahlung dieser Faser in eine Freiraumstrahlung umformbar ist, welche auf ein Patientenauge als ein optisch transparenter und/oder diffusiver, reflektierender Gegenstand **(1,1', 1"; 147; 205)** richtbar ist und ein Referenzarm **(5; 67; 86a, 86b; 157a)** der Messanordnung eine Weglängenvariationseinheit **(39; 55; 61; 71; 89; 161v)** zur Laufzeitänderung hat, wobei im Referenzarm wenigstens zwei einen Laufzeitunterschied hervorrufende Reflektoren **(31a, 31b; 49, 50; 57a, 57b; 69a, 69b; 87a, 87b; 161a-c; 161a-d)** und ein optisches Element **(35; 61)** vorhanden sind, wobei das optische Element die Reflektoren **(31a, 31b; 57a, 57b)** nacheinander mit Messstrahlen belegt, und vorzugsweise die Faser im Messarm mittels einer Faserkupplung auftrennbar ausgebildet ist.

2. Untersuchungs- und/oder Behandlungsplatz nach Anspruch 1, **dadurch gekennzeichnet, dass** die Auswerteeinheit mit der Beobachtungseinheit lösbar verbindbar ist.

3. Untersuchungs- und/oder Behandlungsplatz nach Anspruch 1 oder 2, **gekennzeichnet durch** ein am Patientenmodul **(303)** angeordnetes Anzeigeelement **(315),** welches über eine lösbare elektrische Signalleitung **(316)** mit der Auswerteeinheit **(317)** verbunden ist.

4.  Untersuchungs- und/oder Behandlungsplatz nach einem der Ansprüche 1 bis 3, **dadurch gekennzeichnet, dass** die Beobachtungseinheit mit einem im Patientenmodul **(303)** angeordneten Okular **(323)** und einem Objektiv **(322)** zur Augenbetrachtung ausgebildet ist.

5.  Untersuchungs- und/oder Behandlungsplatz nach einem der Ansprüche 1 bis 3, **dadurch gekennzeichnet, dass** die Beobachtungseinheit ein Bildaufnahmeelement (CCD) **(326a/b)** und eine einen zu betrachtenden Augenbereich auf einem Bildaufnahmeelement **(326a/b)** abbildende Optikeinheit **(325a/b)** hat, wobei Bildaufnahmeelement **(326a/b)** und Optikeinheit **(325a/b)** im Patientenmodul **(303)** angeordnet sind.

6.  Untersuchungs- und/oder Behandlungsplatz nach Anspruch 5, **dadurch gekennzeichnet, dass** Bildaufnahmeelement **(326a/b)** und Optikeinheit **(325a/b)** doppelt und gegeneinander distanziert ausgebildet sind, um eine Stereobetrachtung zu ermöglichen.

7.  Untersuchungs- und/oder Behandlungsplatz nach einem der Ansprüche 1 bis 6, **gekennzeichnet durch** eine Halteeinrichtung für das Patientenmodul, welche als Ausrichteinheit zur Positionierung vor dem Patientenauge ausgebildet ist.

8.  Untersuchungs- und/oder Behandlungsplatz nach einem der Ansprüche 1 bis 7, **gekennzeichnet durch,** eine geometrische Ausbildung des Patientenmoduls in der Grössenordnung eines Kontaktglases, um vor dem Patienten einen nur geringen Raumbereich zu beanspruchen.

9.  Untersuchungs- und/oder Behandlungsplatz nach einem der Ansprüche 1 bis 8, **dadurch gekennzeichnet, dass** der modulare Aufbau derart gewählt ist, dass er lediglich den Platz eines Gerätes beansprucht, aber erlaubt, die Funktionalität unterschiedlicher Einzelgeräte zu erreichen.

10. Untersuchungs- und/oder Behandlungsplatz nach einem der Ansprüche 1 bis 9, **dadurch gekennzeichnet, dass** die Messanordnung und/oder die Beobachtungseinheit mit einer computergestützten Auswerteeinheit zur Auswertung von Messdaten verbunden ist und die Auswerteeinheit über ein Datennetz mit anderen, abrufbare Daten aufweisenden Datenspeichern verbunden ist, um die ermittelten und/oder ausgewerteten Daten mit den anderen Daten verarbeiten zu können.

11. Untersuchungs- und/oder Behandlungsplatz nach einem der Ansprüche 1 bis 10, **dadurch gekennzeichnet, dass** die Reflektoren **(31a, 31b; 49, 50; 57a, 57b; 87a, 87b; 161a-c; 161a-d)** der Messanordnung derart ausgebildet sind, dass sie die auf sie fallende Strahlung in sich selbst reflektieren und tiefenmässig gegeneinander versetzt und miteinander bewegbar sind, um die Laufzeitänderung und den -unterschied zusammen zu erzeugen.

## Claims

1.  Modular assemblable opthalmological examination and/or treatment station with a patient module (303) which can be positioned directly in front of the patient's eye (301), with an evaluation unit (317) which is disposed remotely from the patient module, with an illumination unit (305) which is likewise disposed remotely from the patient module (303), and with at least one fibre coupling part for an optical fibre (304), which comprises a corresponding coupling counterpiece (302), for the detachable connection of the illumination unit (305) which is locally remote from the patient module (303), wherein the patient module (303) has a collimator (310a) which interacts with this optical fibre (304) and with which the radiation of this fibre (304) can be converted into free space radiation (307), with an observation unit (325a/b, 326a/b, 315; 322, 323) which is disposed in the patient module (303), with an optical measuring arrangement (312, 311, 309, 313), which is connected in signal terms to the evaluation unit (317), with at least one further optical fibre (309) and one further collimator (310b), with which the radiation of the at least one further fibre (309) can be converted into free space radiation (312), wherein the further collimator (310b) is disposed in the patient module (303) and the patient module (303) has at least one further fibre coupling part (311) for the detachable coupling of the at least one further fibre (309) belonging to the measuring arrangement (312, 311, 309, 313), wherein the further fibre (309) has a further coupling counterpiece corresponding to the at least one further fibre coupling part (311) and the measuring arrangement has a "Michelson interferometer"-type optical arrangement comprising a short-coherent radiation source (9; 73; 92; 149; 191a-e) and assembled in a substantially fibre-optic manner, a measuring arm (7; 72; 91; 157b) of the measuring arrangement has an optical fibre and a collimator, with which radiation of this fibre can be converted into free space radiation which can be directed at a patient's eye as an optically transparent and/or diffusive, reflecting object (1, 1', 1"; 147; 205), and a reference arm (5; 67; 86a; 86b;

157a) of the measuring arrangement has a path length variation unit (39; 55; 61; 71; 89; 161v) for changing the delay time, wherein at least two reflectors (31a, 31b; 49, 50; 57a, 57b; 69a, 69b; 87a, 87b; 161a-c; 161a-d), which produce a delay time difference, and an optical element (35; 61) are provided in the reference arm, wherein the optical element covers the reflectors (31a, 31b; 57a, 57b) in succession with measuring beams, and the fibre in the measuring arm is preferably formed so as to be splittable by means of a fibre coupling.

2. Examination and/or treatment station according to Claim 1, **characterised in that** the evaluation unit can be connected to the observation unit in a detachable manner.

3. Examination and/or treatment station according to Claim 1 or 2, **characterised by** a display element (315) which is disposed at the patient module (303) and is connected to the evaluation unit (317) via a detachable electrical signal line (316).

4. Examination and/or treatment station according to any one of Claims 1 to 3, **characterised in that** the observation unit is formed with an eyepiece (323), which is disposed in the patient module (303), and a lens (322) for viewing the eye.

5. Examination and/or treatment station according to any one of Claims 1 to 3, **characterised in that** the observation unit has an imaging element (CCD) (326a/b) and an optical unit (325a/b) which forms an image of an eye area to be viewed on an imaging element (326a/b), wherein the imaging element (326a/b) and the optical unit (325 a/b) are disposed in the patient module (303).

6. Examination and/or treatment station according to Claim 5, **characterised in that** the imaging element (326a/b) and the optical unit (325a/b) are formed in duplicate and at a distance from one another in order to permit stereoscopic viewing.

7. Examination and/or treatment station according to any one of Claims 1 to 6, **characterised by** a holding device for the patient module, which device is formed as an alignment unit for positioning in front of the patient's eye.

8. Examination and/or treatment station according to any one of Claims 1 to 7, **characterised by** a geometric formation of the patient module in the order of magnitude of a contact lens in order to occupy just a small spatial area in front of the patient.

9. Examination and/or treatment station according to any one of Claims 1 to 8, **characterised in that** the modular structure is selected such that it only occupies the space of one appliance yet enables the functionality of different individual appliances to be achieved.

10. Examination and/or treatment station according to any one of Claims 1 to 9, **characterised in that** the measuring arrangement and/or the observation unit are/is connected to a computer-assisted evaluation unit for evaluating measurement data and the evaluation unit is connected via a data network to other data stores which comprise retrievable data in order to enable the established and/or evaluated data to be processed with the other data.

11. Examination and/or treatment station according to any one of Claims 1 to 10, **characterised in that** the reflectors (31a, 31b; 49, 50; 57a, 57b; 87a, 87b; 161a-c; 161a-d) of the measuring arrangement are formed such that they reflect the radiation impinging on them into themselves and are staggered in depth and can be moved together in order to produce the delay time change and the delay time difference together.

**Revendications**

1. Poste de traitement et/ou d'examen ophtalmologique pouvant être agencé de façon modulaire avec un module patient (303) pouvant être positionné directement devant l'oeil du patient (301), avec une unité d'évaluation (317) disp6osée à distance du module patient, avec une unité d'éclairage (305) disposée également à distance du module patient (303) et avec au moins un élément d'accouplement de fibre optique pour une fibre optique (304) comprenant une pièce d'accouplement (302) complémentaire correspondante servant à relier de manière amovible l'unité d'éclairage (305 ) éloignée dans l'espace du module patient (303), le module patient (303) possédant un collimateur (310a) agissant conjointement avec cette fibre optique (304), avec lequel le rayonnement de cette fibre (304) peut être converti en rayonnement en espace libre (307), avec une unité d'observation (325a/b, 326a/b, 315 ; 322, 323), qui est disposée dans le module patient (303), avec un dispositif de mesure optique (312, 311, 309, 313) relié par signaux à l'unité d'évaluation (317) avec au moins une autre fibre optique (309) et un autre collimateur (310b), avec lequel le rayonnement de la au moins autre fibre (309) peut être converti en un rayonnement en espace libre (312), auquel cas l'autre collimateur (3 10b) est disposé dans le module patient (303) et le module patient (303) possède au moins un autre élément d'accouplement de fibre optique (311) pour un couplage pouvant être détaché de la au moins une autre fibre (309) appartenant au dispositif de mesure (312, 311, 309, 313), l'autre fibre (309) possédant une autre partie d'accouplement complémentaire correspondant au moins un autre élément d'accouplement de fibre optique (311) et le dispositif de mesure possédant un interféromètre de Michelson; dispositif optique essentiellement à fibre optique comprenant une source de rayonnement (9 ; 73 ; 92 ; 149 ; 191a-e) à courte longueur de cohérence, un bras de mesure (7 ; 72 ; 91 ; 157b) du dispositif de mesure comprenant une fibre optique et un collimateur, avec lequel le rayonnement de cette fibre peut être converti en un rayonnement en espace libre, lequel peut être dirigé sur un oeil du patient comme un objet (1, 1', 1"; 147; 205) réfléchissant, diffus et/ou optiquement transparent et un bras de référence (5 ; 67 ; 86a, 86b ; 157a) du dispositif de mesure possédant une unité de variation de longueur de parcours optique (39 ; 55 ; 61 ; 71 ; 89 ; 161v) pour une modification du temps de propagation, sachant qu'il existe dans le bras de référence au moins deux réflecteurs (31a, 31b ; 49, 50 ; 57a, 57b ; 69a, 69b ; 87a, 87b ; 161a-c ; 161a-d) suscitant une différence de temps de propagation et un élément optique (35 ; 61), sachant que l'élément optique frappe successivement les réflecteurs (31a, 31b ; 57a, 57b) avec des faisceaux de travail, et de préférence la fibre est conçue de manière détachable dans le bras de mesure à l'aide d'un accouplement de fibre optique.

2. Poste de traitement et/ou d'examen selon la revendication 1, **caractérisé en ce que** l'unité d'évaluation peut être raccordée de manière amovible à l'unité d'observation.

3. Poste de traitement et/ou d'examen selon la revendication 1 ou 2, **caractérisé par** un élément d'affichage (315) disposé sur le module patient (303) lequel élément est raccordé à l'unité d'évaluation (317) via une ligne de transmission de signaux (316) électriques amovible.

4. Poste de traitement et/ou d'examen selon l'une des revendications 1 à 3, **caractérisé en ce que** l'unité d'observation est conçue avec un oculaire (323) disposé dans le module patient (303) et un objectif (322) pour observer l'oeil.

5. Poste de traitement et/ou d'examen selon l'une des revendications 1 à 3, **caractérisé en ce que** l'unité d'observation possède un élément de prise de vue (CCD ) (326a/b ) et une unité optique (325a/b) reproduisant une zone de l'oeil à observer sur un élément de prise de vue (326a/b ), l'élément de prise de vue (326a/b) et l'unité d'optique {325a/b) étant disposés dans le module patient (303).

6. Poste de traitement et/ou d'examen selon la revendication 5, **caractérisé en ce que** l'élément de prise de vue (326a/b) et l'unité optique (325a/b) sont conçus de manière double et distancés l'un de l'autre pour permettre une observation stéréo.

7. Poste de traitement et/ou d'examen selon l'une des revendications 1 à 6, **caractérisé par** un dispositif de maintien pour le module patient, lequel est conçu comme une unité d'alignement pour un positionnement devant l'oeil du patient.

8. Poste de traitement et/ou d'examen selon l'une des revendications 1 à 7, **caractérisé par** une configuration géométrique du module patient de l'ordre de grandeur d'un verre de contact pour ne solliciter qu'un espace limité devant le patient.

9. Poste de traitement et/ou d'examen selon l'une des revendications 1 à 8, **caractérisé en ce que** la structure modulaire est choisie de telle manière qu'elle sollicite uniquement la place d'un appareil mais permet d'obtenir la fonctionnalité de différents appareils individuels.

10. Poste de traitement et/ou d'examen selon l'une des revendications 1 à 9, **caractérisé en ce que** le dispositif de mesure et/ou l'unité d'observation est raccordé(e) à une unité d'évaluation assistée par ordinateur visant à évaluer des données de mesure et l'unité d'évaluation est raccordée via un réseau de données à d'autres mémoires de données comprenant des données pouvant être consultées pour pouvoir traiter les données déterminées et/ou évaluées avec les autres données.

11. Poste de traitement et/ou d'examen selon l'une des revendications 1 à 10, **caractérisé en ce que** les réflecteurs (31a, 31b ; 49, 50 ; 57a, 57b ; 87a, 87b ; 161a-c ; 161a-d) du dispositif de mesure sont conçus de telle manière qu'ils reflètent en eux même le rayonnement qui vient les frapper et **en ce qu'**ils sont déplacés en profondeur les uns par rapport aux autres et peuvent être mobiles ensemble, pour générer ensemble la différence de temps de propagation et la modification du temps de propagation.

Fig.1

Fig.2

Fig.3

Fig.5

Fig.6

Fig.10

Fig.4

EP 1 494 575 B1

Fig.7

Fig.8

EP 1 494 575 B1

Fig.9

Fig.11

Fig.12

Fig.13

EP 1 494 575 B1

Fig.14

Fig.15

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0916306 A
- WO 0119303 A
- US 5856883 A
- US 5301101 A
- US 5493109 A
- EP 0932021 A
- WO 9635100 A
- WO 9922198 A